# EUROPEAN PATENT SPECIFICATION

(11) **EP 1 511 767 B1**
(45) Date of publication and mention of the grant of the patent: **22.07.2009**
(21) Application number: 03720299.1
(22) Date of filing: 15.05.2003
(51) Int. Cl.: C07K 14/15, C12N 15/867

(54) **A PURIFIED POLYPEPTIDE, ISOLATED NUCLEIC ACIDS ENCODING SAID POLYPEPTIDE, VECTORS AND USE THEREOF**
GEREINIGTES HÜLLPROTEIN EINES RETROVIRUS, DAFÜR KODIERENDE NUKLEINSÄUREN, VEKTOREN UND DEREN VERWENDUNG
POLYPEPTIDE PURIFIE, ACIDES NUCLEIQUES ISOLES LE CODANT, VECTEURS ET UTILISATION CORRESPONDANTE

(30) Priority: 17.05.2002 DK 200200767
(43) Date of publication of application: 09.03.2005
(73) Proprietor: RetroVec ApS, 8000 Aarhus C (DK)
(72) Inventor: PEDERSEN, Finn, Skou, DK-8210 Århus V. (DK); DUCH, Mogens, Ryttergaard, DK-8140 Risskov (DK); BAHRAMI, Shervin, 8000 Århus C (DK)
(74) Representative: HOEIBERG A/S
(86) International application number: PCT/DK2003/000326
(87) International publication number: WO 2003/097674

(56) References cited:
- EP-A1- 0 742 831
- EP-A2- 1 352 063
- PEDERSEN F S ET AL: "NOVEL LEUKEMOGENIC RETROVIRUSES ISOLATED FROM CELL LINE DERIVED FROM SPONTANEOUS AKR TUMOR" NATURE (LONDON), vol. 292, no. 5819, 1981, pages 167-170, XP002220719 ISSN: 0028-0836 cited in the application
- DAI H Y ET AL: "Multiple sequence elements in the U3 region of the leukemogenic murine retrovirus SL3-2 contribute to cell-dependent gene expression." VIROLOGY. UNITED STATES APR 1990, vol. 175, no. 2, April 1990 (1990-04), pages 581-585, XP008010442 ISSN: 0042-6822 cited in the application
- REIN A ET AL: "DIFFERENT RECOMBINANT MURINE LEUKEMIA VIRUSES USE DIFFERENT CELL SURFACE RECEPTORS" VIROLOGY, RAVEN PRESS, NEW YORK, NY, US, vol. 136, 1984, pages 144-152, XP000569912 ISSN: 0042-6822 cited in the application
- WU B.W. ET AL: 'Identification of Regions in the Moloney Murine Leukemia Virus SU Protein That Tolerate the Insertion of an Integrin-Binding Peptide' VIROLOGY vol. 269, no. 1, 30 March 2000, ACADEMIC PRESS,ORLANDO, US, pages 7 - 17, XP004436321

### SEQUENCE LISTING

<110> Pipeline-Biotech
   Finn Skou Pedersen
   Shervin Bahrami
   Mogens Ryttergaard Duch
<120> A purified retroviral envelope
   polypeptide, isolated nucleic acids encoding said polypeptide, vectors and use thereof
<130> P32118PC01
<150> PA200200767
   <151> 2002-05-17
<160> 2
<170> FastSEQ for Windows Version 4.0
<210> 1
   <211> 1920
   <212> DNA
   <213> Murine Leukaemia Virus
<400> 1
   atggaaggtc cagcgttctc aaaacccctt aaagataaga ttaacccgtg gggcccccta 60
   atagtcctgg gaatcttaat gagggcaaga gtatcagtac aacatgacag ccctcatcag 120
   gtcttcaatg ttacttggag agttaccaac ttaatgacag gacaaacagc taatgctacc 180
   tccctcctgg ggacaatgac cgatgccttt cctaaactgt actttgactt gtgcgattta 240
   ataggggacg actgggatga gactggactc gggtgtcgca ctcccggggg aagaaaaagg 300
   gcaagaatat ttgacttcta tgtttgcccc ggtcacactg tgctagcagg gtgtggaggg 360
   ccgagagagg gctactgtgg caaatgggga tgtgagacca ctggacaggc atactggaag 420
   ccatcatcat catgggacct aatttccctt aagcgaggaa acactcctaa aggccagggc 480
   ccctgttatg attcctcggt ggtctccagt agcgcccagg gtgccacacc ggggggtcga 540
   tgcaaccccc tagtcctaga attcactgac gcgggtaaaa gggccagctg ggacgcctcc 600
   aaagcatggg gactaagact gtaccgatcc acaaggaccg acccggtgac ccggttctct 660
   ttgacccgcc aggtcctcaa tatagggccc cgcgtcccca ttgggcctaa tcccgtgatc 720
   attgaccagt tacccccctc ccgacccgtg cagatcatgc tccccaggcc tcctcagcct 780
   cctccaccag gcgcagcctc tacagtccct gagactgccc caccttccca acaacctggg 840
   acgggagaca ggctgctaaa cctggtaaat ggagcctacc aagctctcaa cctcaccagt 900
   cctgacaaaa cccaagagtg ctggttgtgt ctggtagcgg gaccccccta ctacgaaggg 960
   gttgccgtcc taggtactta ttccaaccat acctctgccc cagctaactg ctccgtggcc 1020
   tcccaacaca agctgaccct gtccgaagtg accggacagg gactctgcgt aggagcagtt 1080
   cccaaaaccc atcaggccct gtgtaatacc acccagaaga cgagcaacgg gtcctactat 1140
   ctggctgctc ccgccgggac catttgggct tgcaacaccg ggctcactcc ctgcctatct 1200
   accactgtgc tcgacctcac caccgattac tgtgtcctgg ttgagctctg gccaaaagtg 1260
   acctaccact cccctggtta tgtttatggc cagtttgaag aaaaaaccaa atataaaaga 1320
   gaacccgtct cactaactct ggccctacta ttaggaggac tcactatggg cggaattgcc 1380
   gccggagtgg gaacagggac taccgcccta gtggccactc agcagttcca acaactccag 1440
   gctgccatgc aggatgacct taaagaagtt gaaaagtcca tcactaatct agaaagatct 1500
   ttgacctcct tgtccgaagt agtgttacag aatcgtagag gcctagatct actattccta 1560
   aaagagggag gtttgtgtgc tgccttaaaa gaagaatgct gtttctatgc cgaccacaca 1620
   ggattggtac gggatagcat ggccaaactt agagaaagat tgagtcagag acaaaaactc 1680
   tttgaatccc aacaagggtg gtttgaaggg ctgtttaaca agtccccttg gttcaccacc 1740
   ctgatatcca ccatcatggg tcccctgata atcctcttgt taattttact ctttgggcct 1800
   tgtattctca atcacctggt ccagtttatc aaagacaggg tttcggtagt gcaggccctg 1860
   gtcctgactc aacaatatca tcaacttaag acaatagaag attgtgaatc acgtgaataa 1920
<210> 2
   <211> 639
   <212> PRT
   <213> Murine Leukaemia Virus
<400> 2
   Met Glu Gly Pro Ala Phe Ser Lys Pro Leu Lys Asp Lys Ile Asn Pro
    1              5                  10                  15
   Trp Gly Pro Leu Ile Val Leu Gly Ile Leu Met Arg Ala Arg Val Ser
               20                  25                  30
   Val Gln His Asp Ser Pro His Gln Val Phe Asn Val Thr Trp Arg Val
           35                  40                  45
   Thr Asn Leu Met Thr Gly Gln Thr Ala Asn Ala Thr Ser Leu Leu Gly
       50                  55                  60
   Thr Met Thr Asp Ala Phe Pro Lys Leu Tyr Phe Asp Leu Cys Asp Leu
   65                  70                  75                  80
   Ile Gly Asp Asp Trp Asp Glu Thr Gly Leu Gly Cys Arg Thr Pro Gly
                   85                  90                  95
   Gly Arg Lys Arg Ala Arg Ile Phe Asp Phe Tyr Val Cys Pro Gly His
               100                 105                 110
   Thr Val Leu Ala Gly Cys Gly Gly Pro Arg Glu Gly Tyr Cys Gly Lys
           115                 120                 125
   Trp Gly Cys Glu Thr Thr Gly Gln Ala Tyr Trp Lys Pro Ser Ser Ser
       130                 135                 140
   Trp Asp Leu Ile Ser Leu Lys Arg Gly Asn Thr Pro Lys Gly Gln Gly
   145                 150                 155                 160
   Pro Cys Tyr Asp Ser Ser Val Val Ser Ser Ser Ala Gln Gly Ala Thr
                   165                 170                 175
   Pro Gly Gly Arg Cys Asn Pro Leu Val Leu Glu Phe Thr Asp Ala Gly
               180                 185                 190
   Lys Arg Ala Ser Trp Asp Ala Ser Lys Ala Trp Gly Leu Arg Leu Tyr
           195                 200                 205
   Arg Ser Thr Arg Thr Asp Pro Val Thr Arg Phe Ser Leu Thr Arg Gln
       210                 215                 220
   Val Leu Asn Ile Gly Pro Arg Val Pro Ile Gly Pro Asn Pro Val Ile
   225                 230                 235                 240
   Ile Asp Gln Leu Pro Pro Ser Arg Pro Val Gln Ile Met Leu Pro Arg
                   245                 250                 255
   Pro Pro Gln Pro Pro Pro Pro Gly Ala Ala Ser Thr Val Pro Glu Thr
               260                 265                 270
   Ala Pro Pro Ser Gln Gln Pro Gly Thr Gly Asp Arg Leu Leu Asn Leu
           275                 280                 285
   Val Asn Gly Ala Tyr Gln Ala Leu Asn Leu Thr Ser Pro Asp Lys Thr
       290                 295                 300
   Gln Glu Cys Trp Leu Cys Leu Val Ala Gly Pro Pro Tyr Tyr Glu Gly
   305                 310                 315                 320
   Val Ala Val Leu Gly Thr Tyr Ser Asn His Thr Ser Ala Pro Ala Asn
                   325                 330                 335
   Cys Ser Val Ala Ser Gln His Lys Leu Thr Leu Ser Glu Val Thr Gly
               340                 345                 350
   Gln Gly Leu Cys Val Gly Ala Val Pro Lys Thr His Gln Ala Leu Cys
           355                 360                 365
   Asn Thr Thr Gln Lys Thr Ser Asn Gly Ser Tyr Tyr Leu Ala Ala Pro
       370                 375                 380
   Ala Gly Thr Ile Trp Ala Cys Asn Thr Gly Leu Thr Pro Cys Leu Ser
   385                 390                 395                 400
   Thr Thr Val Leu Asp Leu Thr Thr Asp Tyr Cys Val Leu Val Glu Leu
                   405                 410                 415
   Trp Pro Lys Val Thr Tyr His Ser Pro Gly Tyr Val Tyr Gly Gln Phe
               420                 425                 430
   Glu Glu Lys Thr Lys Tyr Lys Arg Glu Pro Val Ser Leu Thr Leu Ala
           435                 440                 445
   Leu Leu Leu Gly Gly Leu Thr Met Gly Gly Ile Ala Ala Gly Val Gly
       450                 455                 460
   Thr Gly Thr Thr Ala Leu Val Ala Thr Gln Gln Phe Gln Gln Leu Gln
   465                 470                 475                 480
   Ala Ala Met Gln Asp Asp Leu Lys Glu Val Glu Lys Ser Ile Thr Asn
                   485                 490                 495
   Leu Glu Arg Ser Leu Thr Ser Leu Ser Glu Val Val Leu Gln Asn Arg
               500                 505                 510
   Arg Gly Leu Asp Leu Leu Phe Leu Lys Glu Gly Gly Leu Cys Ala Ala
           515                 520                 525
   Leu Lys Glu Glu Cys Cys Phe Tyr Ala Asp His Thr Gly Leu Val Arg
       530                 535                 540
   Asp Ser Met Ala Lys Leu Arg Glu Arg Leu Ser Gln Arg Gln Lys Leu
   545                 550                 555                 560
   Phe Glu Ser Gln Gln Gly Trp Phe Glu Gly Leu Phe Asn Lys Ser Pro
                   565                 570                 575
   Trp Phe Thr Thr Leu Ile Ser Thr Ile Met Gly Pro Leu Ile Ile Leu
               580                 585                 590
   Leu Leu Ile Leu Leu Phe Gly Pro Cys Ile Leu Asn His Leu Val Gln
           595                 600                 605
   Phe Ile Lys Asp Arg Val Ser Val Val Gln Ala Leu Val Leu Thr Gln
       610                 615                 620
   Gln Tyr His Gln Leu Lys Thr Ile Glu Asp Cys Glu Ser Arg Glu
   625                 630                 635

## Description

### Field of invention

The present invention relates to a purified polypeptide, which is capable of mediating infection of a cell, by use of the polytropic/xenotropic receptor encoded by the Rmc1 locus from a NIH *Swiss* inbred NFS/N mouse for entry, and unable of mediating infection of a cell by use of a human polytropic/xenotropic receptor encoded by the human RMC1 locus.

In a presently preferred embodiment, the present invention relates to an envelope protein from the Murine Leukaemia Virus (MLV) strain SL3-2, which is capable of infecting murine cells through usage of the polytropic receptor encoded by the Rmc1 locus, but lacks the ability of infecting human cells expressing the corresponding xenotropic receptor encoded by the RMC1 locus.

### Background of the invention

Murine Leukaemia viruses are a family of simple retroviruses isolated from laboratory mice. Retroviruses carry their genomes as two copies of a single RNA molecule and the simplest retroviruses contain the *gag, pro, pol* and *env* genes. These genes are found in the same order in all known retroviruses, reflecting the phylogenetic relationship of retroviruses.

The first step in the replication cycle of a retrovirus is its entry into a host cell. The envelope protein (env) is responsible for binding of the retrovirus to a specific cell surface receptor. A retroviral receptor is a membrane integral protein in the plasma membrane of the host cell and as such has a function unrelated to virus infection. From the non-retroviruses capable of pseudotyping, retroviral envelopes that use non-protein receptors are known, e.g. the vesicular stomatitis virus.

Retroviruses can be thought of as a protein-package comprising RNA wrapped in a lipid membrane that contains glycoproteins. The lipid bi-layer is derived from the cell membrane after budding and is thought to be associated with a viral gene product, a peripheral membrane protein called Matrix (MA). Traversing through the lipid bi-layer is another viral gene product, the envelope protein, which consists of two subunits: the transmembrane (TM) and the surface unit (SU). The function of the envelope protein is binding of the virus to its target cell and mediating fusion of the viral and cellular membranes.

Interference studies have defined several different groups of MLVs depending on their receptor usage. Ecotropic viruses utilise the mCAT-1 receptor and are unable to infect non-murine cells. The amphotropic virus uses another receptor and are able to infect cells from a wide variety of organisms including humans. The 10A1 virus is able to bind to two different receptors, both the amphotropic Pit-2, but also the homologous Pit-1 receptor, and has a similarly wide tropism. Finally, the xenotropic viruses utilise the same receptor as the polytropic viruses, but are unable to infect murine cells.

The SL3-2 murine leukaemia virus was isolated from a mouse leukaemia cell line (Pedersen et al., 1981). This virus has a host range similar to mouse ecotropic viruses in that it replicates in mouse cells but not in cell lines such as mink and dog cells. Its ability to infect human cells has not previously been investigated

By RNA oligonucleotide fingerprinting and by receptor interference studies on mouse cells SL3-2 have been related to mouse MCF viruses. However, it does not have the polytropic species host range characteristic of MCF viruses.

The above-mentioned observations were done using a biological isolate, which has not previously been cloned. The SL3-2 virus has been given little attention over the past 15 years.

Ecotropic-and amphotropic MLVs have been widely used as research tools. Ecotropic viruses are usually chosen because of safety concerns, while the amphotropic viruses have the ability to infect human cells. Different packaging cell lines that express the ecotropic or amphotropic envelopes have been designed to fulfil these different requirements.

Retroviral integration can activate genes in the vicinity of the integration site. In this way, retroviruses have been used to identify oncogenes since activation of these genes result in tumour growth. In much the same way the integration of a provirus can disrupt the expression of genes, hence inactivation of a tumour suppressor gene may contribute to tumour formation. A high number of integration's are desirable in such studies since not all integration's result in tumour generation and multiple hits are required. Very few integration events are expected to be near oncogene or tumour suppressor genes. Tumour formation might also involve multiple gene regulations.

Retroviral infections usually result in a single integration event since the envelope protein blocks receptors on an infected cell. This is the basis of the superinfection resistance (also called interference) phenomenon in which a virus-infected cell shows resistance to superinfection by viruses, which utilise the same receptor for entry. Thus, use of viruses with different receptor usage increases the number of integration events. Entry by different receptors may even provide access to retroviral disease induction in different mouse tissues.

The integration mechanism of retroviruses can be used to introduce any DNA sequence into a host genome, if the appropriate *cis* elements of the retroviral genome are maintained in the transducing vector and the DNA sequence can be encompassed in the vector (less than 9000 bp). Therefore retroviral vectors are attractive tools for gene therapy. Most simple retroviral receptors are found on many different cell types of the same species. That is why vector systems utilising wild type envelopes from simple retroviruses cannot be used to introduce genes in a selective manner into specific cells/tissues. Retargeting envelope proteins remains an elusive goal.

While many attempts have been done to change the tropism of viruses by introducing novel binding domains such as single chain antibodies into envelope proteins, most have been unsuccessful. The chimeric envelopes usually obtain the ability to bind to the intended target, but loose their fusion activity. In some cases expression of the wild type envelope is necessary to achieve any infection of the target cells. Ecotropic envelopes have been used in most of such studies since they lack the ability to infect human cells. Chimeric envelope based on an ecotropic envelope is unlikely to cause secondary, unintended infections.

Within the well-characterised group of gamma-retroviruses, other envelopes that do not infect human cells have not been available. So far, there has not been any explanation for the species tropism of gamma-retroviruses. Any research into this area that results in methods for alterations of species tropism may have important consequences for designing vectors as tools for human gene therapy.

### Detailed description of the invention

The present invention relates to a polypeptide as described in the appended claims capable of mediating infection of a cell, by use of the polytropic/xenotropic receptor encoded by the Rmc1 locus of the NIH *Swiss* inbred NFS/N mouse for entry, and unable of mediating infection of a cell by use of a human polytropic/xenotropic receptor encoded by the human RCM1 locus. The origin of the polypeptide described by the present inventors is per se not species specific, but could be derived from polypeptides originating from e.g. vira, plasmids, Prokaryote, Eukaryote, Archaea, or Mammalia.

The present invention especially relates to a new purified retroviral envelope polypeptide as described in the appended claims originating from the Murine Leukaemia Virus (MLV) SL3-2. As shown by the present inventors, this new polypeptide is capable of mediating infection of a cell, by use of the poiytroplc/xenotropic receptor encoded by the Rmc1 locus of the NIH *Swiss* inbred NFS/N mouse for entry, and unable of mediating infection of a cell by use of a human polytropic/xenotropic receptor encoded by the human RCM1 locus.

The present invention also relates to a new defined region within said polypeptide named VR3. Surprisingly, changing specific amino acids within the VR3 region can alter the host tropism of SL3-2. The present inventors has pin-pointed exactly which amino acid that is essential for this host tropism shift and thus relates to substitutions of a amino acid within the VR3 region.

The present invention further relates to cells, capable of displaying, vectors comprising, packaging cells comprising, the envelopes as described in the appended claims. Further, the use of said envelopes is as described in the appended claims.

### The SL3-2 envelope polypeptide

The SL3-2 envelope polypeptide described in the present application specifically uses the polytroplc/xenotropic receptor encoded by the Rmc1 locus of the NIH *Swiss* inbred NFS/N mouse for entry. Thus, the SL3-2 envelope polypeptide of the present invention is unable to enter a cell by use of the human homolog of said receptor. The polytroplc/xenotropic receptor encoded by the Rmc1 locus originally derived from a NIH *Swiss* inbred NFS/N mouse, can be transferred to any cell by an expression vector comprising said receptor and thereby enabling entry. Thus, one embodiment of the present invention relates to a purified retroviral envelope polypeptide, capable of mediating infection of a cell, by use of the polytropic/xenotropic receptor encoded by the Rmc1 locus isolated from a NIH *Swiss* inbred NFS/N mouse for entry, and unable of mediating infection of a cell by use of a human polytropic/xenotropic receptor encoded by the human RMC1 locus.

Another embodiment of the present invention relates to a purified retroviral envelope polypeptide, capable of mediating infection of a cell derived from *Mus musculus* by use of the polytropic/xenotropic receptor encoded by the Rmc1 locus isolated from a NIH *Swiss* inbred NFS/N mouse for entry, and unable of mediating infection of a human cell comprising a human polytropic/xenotroplc receptor encoded by the human RMC1 locus.

A presently preferred embodiment of the present invention relates to a purified murine retroviral envelope polypeptide, capable of mediating infecting of a cell derived from *Mus musculus,* by use of the polytropic/xenotropic receptor encoded by the Rmc1 locus isolated from e.g. a NIH *Swiss* inbred NFS/N mouse for entry, and unable of mediating infection of a human cell comprising a human polytropic/xenotropic receptor encoded by the human RMC1 locus.

In the present context the term "unable of mediating infection of a human cell" refers to virus particles comprising the SL3-2 envelope, not capable of infecting human cells, while capable of infecting mouse NIH -3T3 cells. In a laboratory test this term can further be defined as an isolate of virus particles comprising said SL3-2 envelope providing a titer below 10² infectious units per ml on the human cells, while at the same time providing a titer above 10⁵ infectious units per ml on the murine cells, or as an isolate of viruses comprising said SL3-2 envelope where the difference in titer between the permissive (in casu laboratory mouse cells) and the non-permissive cells (in casu human cells) is greater than 10³ infectious units per ml.

### Fv-1 restriction

Murine cells have several restrictions to retrovirus infection. One such restriction is the Fv-1 restriction that operates after binding to the receptor and internalisation of the virus and before integration of the virus into the host genome. This restriction is mediated through an endogenous *gag* like sequence that interacts with the *gag* protein CA in the virus core particle, disabling the normal integration process of the virus. A cell can be either Fv-1^{n/n}, Fv-1^{b/b}, Fv-1^{n/b}, or Fv-1^{o/o}. The virus can be either N-, B-, or NB-tropic.

Fv-1 is a co-dominant trait, as Fv-1^{n/b} animals can not be infected by neither N- nor B-tropic viruses, only by NB-tropic viruses. Fv-1^{n/n} animals can be infected by N-tropic viruses and vice versa Fv-1^{b/b} can be infected by B-tropic viruses. The restriction is not 100%, rather the titer is reduced between 50-1000 fold in a Fv-1 permissive contra a Fv-1 non-permissive cells.

Almost all or all MCF viruses described are N-tropic in host range (Hartley et al., 1977) meaning that these viruses can infect cells that are Fv-1^{n/n}, but not Fv-1^{b/b}.

Human cells have a restriction very similar to this Fv-1 restriction (Towers et al., 1999)(Aagaard et al., 2002). All human cells tested so far, show a restriction that prevents N-tropic virus infection resulting in a general restriction of human cells to wild type infection of MCF viruses, even if initial attachment and fusion of the virus particle is successful.

To assay if a given virus can or cannot utilise the human polytropic/xenotropic receptor encoded by the RMC1 locus, the receptor has to be expressed in a cell permissive for N-tropic virus infection or the envelope pseudo-typed with a Gag protein of either B- or NB-tropic virus.

The SL3-2 envelope described by the present inventors, has been characterised in a mini-virus setting, where the Gag/Pol proteins were provided by the Moloney-MLV. This virus is of NB-tropic origin and can thus mediate infection of human cells.

As described by the present inventors, the SL3-2 envelope has been cloned from genomic DNA of NIH 3T3 cells infected with SL3-2 by PCR amplification. The amplified PCR fragment was subsequently cloned and sequenced.

Thus, one embodiment of the present invention relates to a purified envelope polypeptide having an amino acid sequence which is at least 94% identical to the amino acid sequence shown in SEQ ID NO:2, or a fragment of said amino acid sequence that is at least 94% identical to the amino acid sequence shown in SEQ ID NO:2, such as 94.5% identical, 95% identical, 95.5% identical, 96% identical, 96.5% identical, 97% identical, 97.5% identical, 98% identical, 98.5% identical, 99% identical, or 99.5 % identical.

The similarity between two nucleic acid sequences, or two amino acid sequences, is expressed in terms of the similarity between the sequences, otherwise referred to as sequence identity. Sequence identity is frequently measured in terms of percentage identity (or similarity or homology); the higher the percentage, the more similar the two sequences will be.

Methods of alignment of sequences for comparison are well known in the art. Various programs and alignment algorithms are described and present a detailed consideration of sequence alignment methods and homology calculations, such as VECTOR NTI.

The NCBI Basic Local Alignment Search Tool (BLAST) is available from several sources, including the National Center for Biotechnology Information (NBCI, Bethesda, Md.) and on the Internet, for use in connection with the sequence analysis programs blastp, blastn, blastx, tblastn and tblastx. It can be accessed at http://www.ncbi.nim.nih.gov/BLAST/. A description of how to determine sequence identity using this program is available at http://www.ncbi.nim.nih.gov/BLAST/blast_help.html.

Homologs of the disclosed polypeptides are typically characterised by possession of at least 94% sequence identity counted over the full length alignment with the disclosed amino acid sequence using the NCBI Basic Blast 2.0, gapped blastp with databases such as the nr or swissprot database. Alternatively, one may manually align the sequences and count the number of identical amino acids. This number divided by the total number of amino acids in your sequence multiplied by 100 results in the percent identity.

In a presently preferred embodiment the envelope polypeptide has the amino acid sequence shown in SEQ ID NO:2.

The SL3-2 envelope shows quite high homology with the envelope protein of MCF-247 polytropic MLV and the biological effect of the polypeptide envelope described by the present inventors, mediation of infection through a specific receptor, is generated solely by the amino acid sequence of said envelope. As known to a person skilled in the art, a codon of an amino acid can be generated by various nucleic acid sequences, due to the degeneracy of the genetic code thus the present invention relates to all isolated nucleic acid sequences capable of encoding the envelope polypeptide sequences within the scope of the present invention.

In a presently preferred embodiment, said nucleic acid sequence is capable of encoding an amino acid sequence that is at least 94% identical to the amino acid sequence as shown in SEQ ID NO:2, such as 94.5% identical, 95% identical 95.5% identical, 96% identical, 96.5% identical, 97% identical, 97.5% identical, 98% identical, 98.5% identical, 99% identical, or 99.5 % identical.

In a presently most preferred embodiment of the present invention, the isolated nucleic acid sequence of SL3-2 is as shown in SEQ ID NO:1.

### The mutated SL3-2 envelope polypeptide

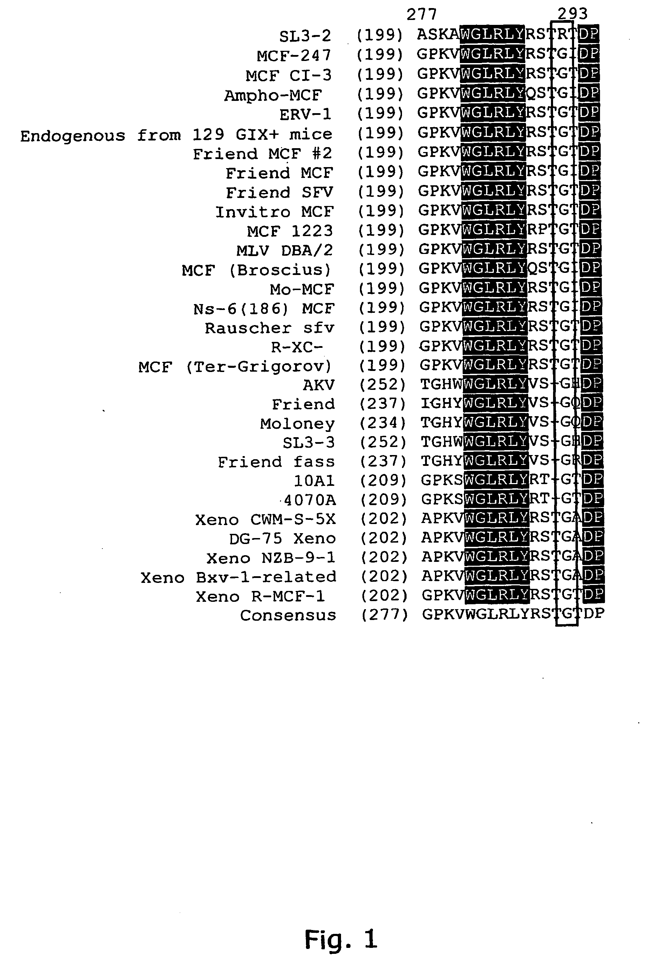

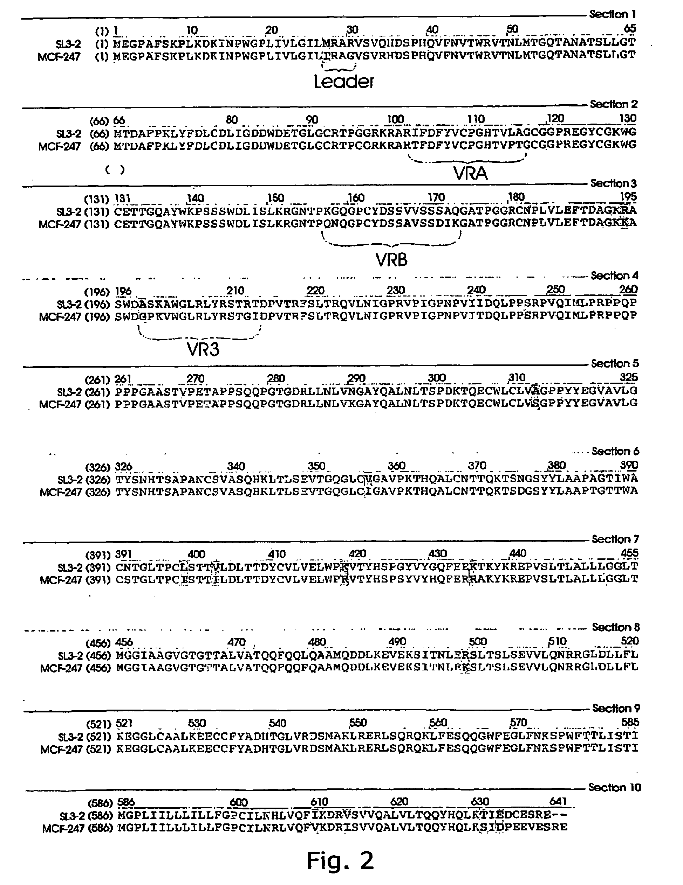

In a sequence alignment between SL3-2 and MCF-247, three regions display differences in the amino acid sequence, as described in example 3 and figure 1 and 2. Two of these regions correspond to parts of the variable VRA and VRB regions, whereas the third is a 15 amino acids long stretch upstream of the proline rich region. The present inventors have named this region VR3.

### VR3 region

Further, a sequence alignment of MLVs from different sub-families show conserved amino acids at positions 203-208 WGLRLY and at positions 214-215 DP based on SL3-2 sequence, thus defining a 13 amino acid stretch of SEQ ID NO:2.

In the present context, the term "VR3 region" comprises all of the amino acids found between the residue found at two positions after the conserved tryptophan 197 and the residue before the conserved aspartic acid 214 (according to the sequence shown in SEQ ID NO:2) including these two positions.

The SL3-2 wild type envelope described in the present application contains an arginine residue in the amino acid position 212, see SEQ ID NO:2. The present application demonstrates that this amino acid is a most crucial amino acid, which appears to be responsible for the limited tropism of the wild type SL3-2 envelope.

To further investigate the determinants of the differences in tropism between SL3-2 and MCF-247, chimeras were created in which the 15 amino acid VR3 region in SL3-2 was replaced by corresponding part of MCF-247, by use of an overlap extension method. By replacing the VR3 region, the SL3-2 envelope was now capable of infecting a human cell by use of the human polytropic/xenotropic receptor encoded by the RMC1 locus for entry. This shows that the tropism lies within the VR3 region of the SL3-2 virus.

Further analysis showed that the merely a replacement of the argenine in position 212 of the VR3 region with a glycine could mediate infection of a human cell by the human polytropic/xenotropic receptor encoded by the RMC1 locus. Glycine is the wild type amino acid found at the corresponding position in all other MLV strains. Thus, the present invention relates to a VR3 region with an arginine within said region at the corresponding position 212, but also relates to the replacement of said arginine.

"Corresponding position" in the present context relates to two amino acids before the conserved aspartic acid at position 214 in SL3-2 sequence.

One embodiment of the present invention relates to a purified envelope polypeptide wherein said polypeptide includes at least one substitution in the VR3 region. In a presently preferred embodiment, the present invention relates to a purified envelope polypeptide, wherein said mutation is at position 212 in SEQ ID NO:2.

Glycine is the smallest amino acid with no significant side chain. It is possible that replacing this residue with an arginine, which has a much larger side chain, confers steric problems for binding of the SL3-2 envelope to the human polytropic receptor. If so, replacing the glycine with other large amino acids is expected to result in changes in tropism. Examples of other substitutions which are likely to provide the same effect are alanine, asparagine, aspartic acid, cysteine, glutamic acid, phenylalanine, glycine, histidine, isoleucine, lysine, leucine, methionine, proline, glutamine, serine, threonine, valine, tryptophan or tyrosine.

Further randomised library selection at amino acid position 212-213 in fact identifies methionine-212 as a mediator of efficient infection, see example 6 and tables 7-8.

Arginine is a basic amino acid and is positively charged in the physiological pH. The positively charged arginine in the wild type SL3-2 could enhance binding to the negatively charged glutamic acid in NIH 3T3 murine receptor while interfering with the positive charge of lysine found in receptors of other species.

In one preferred embodiment the mutation at position 212 in SL3-2 changes the arginine to glycine. In another more preferred embodiment the mutation at position 212 in SL3-2 results in a methionine.

SL3-2 is the only MLV with an arginine at position 212. The conservation of glycine at this position in different sub-families of MLV seems to point toward a receptor independent function for this region of envelope protein. The entry mechanism of MLV envelopes is largely unknown.

The present application describes a new surprising effect by a single mutation in the VR3 region of SL3-2 envelope polypeptides, thus a person skilled in the art would be able to determine if further mutations could further alter and/or enhance the entry properties of these envelope polypeptides. Thus, another embodiment of the present invention relates to a purified envelope polypeptide wherein said polypeptide includes at least one substitution in the VR3 region, such as 1, 2, 3, 4, 5 or 6 substitutions in the VR3 region, see figure 1 and 2.

The present invention further relates to a purified mutated envelope polypeptide, wherein said at least one substitution alters the host tropism of a virus or an infectious particle displaying said polypeptide. A presently preferred embodiment of the present invention relates to a, purified envelope polypeptide, wherein said purified polypeptide is a retroviral envelope polypeptide originally of murine origin capable of mediating infection of a human cell with a higher titer than a xenotropic or polytropic virus in a comparable system.

In the present context, an infectious particle is a particle comprising of various viral components that are assembled *in vitro* to a particle capable of mediating transduction (attachment fusion, reverse transcription and integration) of a retroviral vector. One embodiment of the present invention relates to a mutated SL3-2 purified envelope polypeptide capable of mediating a higher infectivity in human cells than other MLV's, such as, but not limited to MCF-247, MCF-13 and X-MLV (NZB) viruses.

In the present context, the term " higher infectivity" relates to any situation wherein the infection titre of one envelope is at least 2 fold higher than the other envelope with which it is compared.

### Further modifications to envelope

Envelope proteins bind to cellular receptors and mediate infection by fusing the viral and cellular membranes. Retroviruses, even very similar ones, utilise different cell surface receptors. Receptor binding has been mapped to the N-terminal domain of SU subunit of envelope protein. Accordingly, the largest differences in amino acid sequence among MLV sub-families are found in the N-terminal receptor-binding domain. The receptors used by many retroviruses are housekeeping proteins such as amino acid transporters. Therefore these viruses are not tissue specific and vectors using such envelopes will be able to infect the majority of the cells In an organism.

In many of the predicted therapeutic applications of retroviral vectors, targeting specific cell types/tissues is vital and therefore changing receptor usage of envelope proteins is one of the major goals of the retroviral envelope research. The fact that related envelope proteins can utilise different receptors has fuelled the idea that the tropism of envelopes can be changed by modifying the receptor binding domain by e.g. inserting non-viral sequences. This modification could be in form of inserting peptide ligands or single chain antibodies against specific cell surface molecules. Post-translational chemical modification of envelope proteins by attaching ligand molecules to envelope proteins might achieve the same goal. Targeting specific cell types is possible in this way.

Several functional chimeric envelopes have already been described but none of these can mediate transduction at efficiencies comparable to the efficiencies obtained with wild type envelope proteins. The described functional chimeric MLV-envelopes can be divided into two groups. The first group has the heterologous ligand inserted in the N-terminal of the SU-protein and can mediate transduction without co-expression of wild type envelope, whereas the other group has the ligand inserted internally in SU and is dependent of co-expressed wild type envelope. Peptide linkers and a single chain antibody specific for the human major histocompatibility complex class I (MHC-I) molecule have e.g. been inserted at four internal positions in Akv-*env*.

The first attempts to direct virus particles towards receptors not normally recognised by retroviruses were done by antibody-bridging and by usage of chemical modifications. By cross-linking monoclonal antibodies against SU and the transferring receptor with a sheep anti-mouse kappa light chain antibody binding of the virus to human HEp2 cells, and subsequent internalisation was shown. However, internalisation of the virus by this infection route was not followed by establishment of the proviral state.

Others used a similar approach to target the attachment of ecotropic viruses by streptavidin bridging biotinylated antibodies against SU and against specific membrane markers expressed on human cells. By this method human cells expressing MHC class I, MHC class II, epidermal growth factor and Insulin were successfully infected, whereas this method did not prove feasible for promoting infection of cells expressing transferrin, high density lipoprotein and galactose receptors.

Also, chemically coupled galactose residues to ecotropic Env, making the virus particles capable of infecting human hepatoma cells through the asialoglycoprotein receptor, have been tried.

Infection of human cells by an ecotropic virus displaying chimeric-envelope proteins on the surface of the virion is also known to a person skilled in the art. This can be achieved by e.g. substituting a part of MoMLV SU with a sequence encoding the erythropoietin hormone (EPO), insertion of a sequence encoding human heregulin for infection of human breast cancer cells overexpressing the human epidermal growth factor receptor, substitution of an internal fragment of SU with a single-chain variable fragment (ScFv) derived from a monoclonal antibody recognising the human low density lipoprotein receptor which gave a chimeric envelope capable of infecting human cells.

In these reports with chimeric envelopes, targeted infection was only obtained when wild type *env* was co-expressed with the chimeric construct (from the ψ2 packaging cell line). This indicates that functional domains are contained within the ecotropic envelope, which is necessary for mediating infection beyond the point of receptor binding.

Various potential ligands have been used e.g. a ScFv against a specific hapten between amino acid 6 and 7 (position +7) in MoMLV SU, the amphotropic-MLV binding domain (208 amino acids), the polypeptide able to bind epidermal growth factor receptor (EGFR)(53 amino acids of EGF), collagen-binding properties, and a ligand (70 amino acids) recognising ErbB-3 and ErbB-4. The obtained targeting efficiencies with chimeric envelopes reported until now are considerably lower than the efficiencies obtained with wild type envelopes. The reasons for these low transduction efficiencies of target cells are probably diverse, including the choice of insertion site, stability of the chimeric envelope protein, the tertiary protein structure and the choice of target cells. Furthermore, the choice of ligand is probably also very important for obtaining infection, as several chimeric envelopes have failed to promote infection. One more positive example relates to insertion of a short nondisruptive peptide (RDG) known to bind to several integrins displayed on the surface of cells (Golan T3 and Green-MR, 2002).

The above-described examples all utilised the ecotropic envelope. One advantage of using this envelope is that it is restricted in infecting human cells as the surface protein part of the envelope does not recognise a human receptor. The concept is that if the envelope can be engineered to bind to a human receptor by inserting a heterologous sequence in the envelope mediating this binding, the otherwise intact fusogenic properties of the envelope would mediate the fusion. Like the ecotropic envelope the SL3-2 envelope cannot mediate infection of human cells. But by mutating a single amino acid this tropism can be changed making the SL3-2 envelope a much better scaffold than the ecotropic MLV envelope for genetic engineering of chimeric envelopes with a redirected specificity for a new cognate receptor

Thus, a presently preferred embodiment of the present invention relates to a purified envelope polypeptide further comprising an inserted non-viral sequence, capable of redirecting the target cell specificity, by the resultant chimeric envelope.

Once the inserted non-viral sequence is placed within the envelope, a person skilled in the art would be capable of mutating essential amino acids of within said sequences similar to e.g. the VR3 region. Thus the present invention a purified envelope polypeptide comprising an inserted non-viral sequence, wherein the chimeric envelope further contains secondary mutations, enabling activation of the fusiogenic properties of said chimeric envelope, by binding of the receptor target. In a presently preferred embodiment of the present invention said inserted sequence is a single chain antibody or e.g. a peptide ligand. Another embodiment of the present invention relates to a purified envelope according to the present invention further comprising a chemical modification of said envelope. In a presently preferred embodiment said chemical modification enhance and/or alters the host tropism.

One embodiment relates to a recombinant mammalian cell capable of displaying an envelope polypeptide according to the present invention.

The biological effect of any of the polypeptide envelopes described by the present inventors, is mediation of infection through a receptor. This mediation is generated solely by the polypeptide having the amino acid sequence of said envelope polypeptide. As known to a person skilled in the art, a codon of an amino acid can be generated various nucleic acid sequences, thus the present invention relates to all isolated nucleic acid sequences capable of encoding an envelope polypeptide having an amino acid sequence as described in the present application. Thus, the present invention relates to an isolated nucleic acid sequence encoding any of the envelope polypeptides described in the present invention.

In a presently preferred embodiment said isolated nucleic add sequence is the nucleic acid sequence shown in SEQ ID NO:1 comprising the mutation at position 212, compared to the SL3-2 envelope polypeptide.

### Vectors and packaging cells

A recombinant mammalian expression vector in he present context comprises all vectors capable of directing expression of any given envelope by directing expression of vector DNA into RNA, poly-adenylation of said RNA, splicing of said RNA, if necessary, export out of the nucleus of said RNA, and finally translation of said RNA outside of the nucleus.

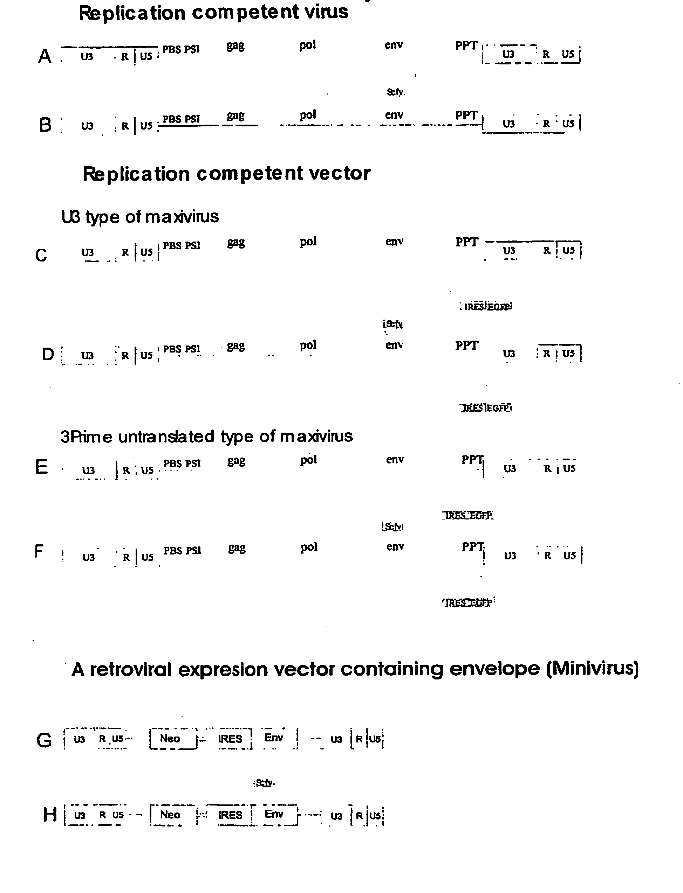

A replication competent retrovirus further comprises, all genes necessary for replication of a retrovirus, and for RNA being exported out of the cell and packaged in proteins expressed by said proteins. Said RNA further comprises all RNA and DNA elements necessary for said RNA to be reverse transcribed into double stranded DNA and integrated into the host genome, as exemplified in figure 3 panel A. In panel B this replication competent retrovirus further comprises an ScFv or any heterologous peptide inserted into the envelope gene for redirection of host cell tropism. Only the ScFv is depicted in figure 3, but other insertions such as an RGD peptide could be similarly useful.

The exemplified replication competent retroviral vector further comprises a replication competent virus where a heterologous gene is being expressed from a position in the U3 region of the virus, panel C and D, or from a position in the 3 prime untranslated region downstream of the envelope and upstream of the downstream LTR, panel E and F. Said replication competent vectors can further be redirected in host cell tropism by insertion of an ScFv or any heterologous peptide in the envelopes, panel D and F. Only the ScFv is depicted in figure 3. Based upon this example, the text of the present application and common knowledge of a person of ordinary skill in the art will be able to make other useful embodiments.

The term "a retroviral expression vector" comprises a retroviral vector being capable of transcribed into RNA and capable of being packaged into a retroviral particle, reverse transcribed into double stranded DNA and inserted into the host genome by the retroviral enzymatic machinery. For translation of said envelope an internal ribosome entry site (IRES) has been inserted upstream of the envelope in the exemplified retroviral expression vector, panel G and H. The host cell tropism of said retrovirus can further be redirected by inserting an ScVf or any heterologous peptide in the envelope, panel H. Only the ScFv is depicted in figure 3, but other inserts could be similar useful.

One embodiment of the present invention relates to a replication competent retrovirus or any of the vectors described in the present application, comprising a purified retroviral envelope polypeptide, capable of mediating infection of a cell, by use of the polytropic/xenotropic receptor encoded by the Rmc1 locus Isolated from a NIH *Swiss* inbred NFS/N mouse for entry, and unable of mediating infecting of a cell by use of a human polytropic/xenotropic receptor encoded by the RMC1 locus.

Another presently preferred embodiment relates to a replication competent retrovirus or any of the vectors described in the present application, comprising a purified retroviral envelope polypeptide, capable of mediating infecting of a cell derived from *Mus musculus,* by use of the polytropic/xenotroplc receptor encoded by the Rmc1 locus isolated from a NIH *Swiss* inbreed NFS/N mouse for entry, and unable of mediating infection of a human cell comprising a human polytropic/xenotropic receptor encoded by the RMC1 locus.

Further, the present invention also relates to a replication competent retrovirus or any of the vectors described in the present application, comprising a purified murine retroviral envelope polypeptide, capable of mediating infection of a cell derived from *Mus musculus,* by use of the polytropic/xenotropic receptor encoded by the Rmc1 locus isolated from a NIH *Swiss* inbreed NFS/N mouse for entry, and unable of mediating infection of a human cell comprising a human polytroplc/xenotroplc receptor encoded by the RMC1 locus.

In a presently preferred embodiment the present invention relates to a replication competent retrovirus or any of the vectors described in the present invention, comprising a purified envelope polypeptide having an amino acid sequence that is at least 94 % identical to an amino acid sequence such as shown in SEQ ID NO: 2 94 %, or a fragment of said amino acid sequence that is at least 94 % identical to a fragment of an amino acid sequence such as shown in SEQ ID NO: 2 94 %.

Another presently preferred embodiment of the present invention relates to a replication competent retrovirus or any of the vectors described in the present application, comprising a purified envelope polypeptide, wherein said polypeptide includes at least one substitution in the VR3 region. In a presently preferred embodiment, said mutation in the VR3 region is at position 212, as shown in SEQ ID NO: 2. In a presently most preferred embodiment of the present application said mutation alters the host tropism of a virus or an infectious particle displaying said polypeptide.

A further presently preferred embodiment of the present invention relates to a replication competent retrovirus or any of the vectors described in the present application, comprising a purified envelope polypeptide, wherein said purified polypeptide is a murine retroviral envelope polypeptide capable of mediating infection of a human cell.

It is an object of the present invention to provide a replication competent retrovirus or any of the vectors described in the present application, comprising a mutated purified SL3-2 envelope polypeptide which is capable of mediating a higher infectivity in human cells than MCF-247, MCF-13 and X-MLV (NZB) viruses.

Another object of the present invention is to provide a replication competent retrovirus or any of the vectors described in the present application, comprising any of the purified retroviral envelopes mentioned in the present application and further comprising an inserted non-viral sequence capable of redirecting the target cell specificity, by the resultant chimeric envelope. Another object of the present invention is to provide a replication competent retrovirus or any of the vectors described in the present application comprising a chimeric envelope, wherein said chimeric envelope further contains secondary mutations, enabling activation of the fusiogenic properties of said chimeric envelope, by binding to the receptor target.

A presently preferred object of the present invention is to provide a replication competent retrovirus or any of the vectors described in the present application, comprising any of the purified retroviral envelopes mentioned in the present application and further comprising an inserted non-viral sequence, wherein said inserted sequence is a single chain antibody.

Another object of the present invention is to provide a replication competent retrovirus or any of the vectors described in the present application comprising any of the purified retroviral envelopes mentioned in the present application and further comprising a chemical modification of said envelope.

A preferred object of the present invention is to provide a replication competent retrovirus or any of the vectors described in the present application, comprising any of the purified retroviral envelopes mentioned in the present application and further comprising a chemical modification of said envelope, wherein said chemical modification enhances and/or alters the host tropism.

A particular embodiment of the present invention relates to any of the replication competent vectors described in the present application and further comprising a heterologous translation cassette.

A presently preferred particular embodiment relates to a replication competent vector comprising a heterologous translation cassette, wherein said heterologous translation cassette comprises an IRES-gene element.

Another particular embodiment of the present invention relates to a vector according to the present invention further comprising at least one heterologous gene to be expressed.

In a presently preferred particular embodiment, the present application relates to a vector according to the present invention further comprising at least one heterologous gene to be expressed, wherein said expression is directed by a IRES-element.

Retroviral genomes can be divided into two different functional parts. The protein coding genes are not necessary as *cis* elements (RNA or DNA in the present context) sequences for viral replication cycle. Only their encoded proteins have a role to play. In contrast there are a few other elements in a retroviral genome that are necessary for viral replication. The packaging signal is one such element and is necessary for packaging of the viral genome into budding virions.

A retroviral vector technology is based on separation of these *cis* and *trans* elements and has two major elements: a vector and a packaging cell line. A simple vector is a viral genome in which the protein-coding genes are replaced by a heterologous sequence. The packaging cell line is engineered to produce the viral Gag, Pol and Env proteins from constructs that lack the packaging signal (to prevent them from being taken up by budding virions). Thus, when a vector is inserted into a packaging cell line, it will be packaged into budding virions and can be transferred into target cells.

MLV based packaging cells are widespread tools for research. Packaging cells based on ecotropic viruses have the advantage of being harmless to humans and are used in blo-safety level 1 laboratories. Since all ecotropic envelopes use the same receptor, superinfection resistance might necessitate the use of virions other than ecotropic, if infection of an ecotropic based packaging cell line is desired. So far, the choices in these situations have been an amphotropic based cell line despite the greater safety concerns involved.

The term "superinfection resistance" is used in the present context to describe the fact that viral infected cells express the viral envelope protein on their surface. Here the envelope protein and the cellular receptor interact with one another and receptors are thereby masked from external viral particles. Thereby an infected cell cannot be infected again by the same virus genus or by any other virus that use the same receptor for entry. This phenomenon is also called interference and is a convenient means of determining if two different viruses utilise the same receptor.

A packaging cell line based upon the envelope polypeptides of the present invention can replace the amphotropic cell lines and in the same time, have the benefit of higher safety. A packaging cell line using the envelope polypeptides of the present invention may also be more effective in infecting murine cell types with low expression of the ecotropic receptor.

One embodiment of the present invention relates to a packaging cell construct comprising a recombinant mammalian expression vector comprising a nucleic acid coding for a polypeptide envelope as described in the present application, and a non-viral or viral promoter and poly-adenylation signals.

Another embodiment of the present invention relates to use of any of the vectors according to the present invention for the generation of a packaging cell.

The mini-virus system described below is a variation of the retroviral vector technology. The main difference is that in the mini-virus system the envelope protein is expressed from the vector instead of the cell line. The complementing cell line for this vector contains only the viral *gag* and *pol* genes and is referred to as a semi-packaging cell line.

One embodiment of the present invention relates to use of a vector (mini-virus) in a cell constitutively, or inducible, or in any other way expressing the gag/pol genes of simple retroviruses.

### Disease models

By including into a replication competent virus an additional gene, an oncogene, a proto-oncogene or a dominant-negative form of a tumor-suppressor gene, new MLV-based models for multistep oncogenesis have been established. This strategy aims at analysing co-operating events resulting from the introduced gene and the effect on host genes caused by proviral insertion. The first integration of a provirus harbouring a gene with oncogenic activity will provide two hits, one resulting from the introduced gene and one from the effect at the site of integration. Such models resemble transgenic and knockout mouse models where one activating event is introduced in the germ-line. However, the results may differ because the gene with oncogenic activity will not be present in the embryo and will only enter cells together with the virus.

In general, the murine leukaemia viruses (MLVs) constitute a polymorphic group of non-acute transforming retroviruses. The pattern of disease induction varies among individual strains and mutants and also depends upon the specific laboratory mouse strain.

Although the overall pathogenesis of MLV infections is still poorly understood, insertional mutagenesis of critical host genes by proviral integration has been established to be of importance for induction of lymphomas and leukaemia's. Differences in long terminal repeat (LTR) enhancer sequences associated with binding of specific host transcription factors contribute to variation in the pathogenic potency and specificity among MLV variants and mutants. However, differences in other parts of the genome can also be of importance e.g. the envelope. Retroviral models have the advantage that critical host genes can be identified through tagging by integrated proviruses or by means of the interaction of their products with parts of the viral machinery. Rapid advances in genome analysis shorten the path to critical host genes of the animal model and to their human counterparts.

Thus, it is an object of preferred embodiments of the present invention to provide retroviruses expressing a retroviral envelope polypeptide of the invention as these viruses may infect a different population of target cells than do the ecotropic envelopes. By targeting a different subset of cells these viruses may invoke different sets of critical host genes and thereby further exploit the information from proviral integration sites in available tumours towards the identification and functional characterisation of critical host genes involved in oncogenesis.

The advantages of using a retroviral envelope polypeptide having the properties of a wild type SL3-2 envelope as compared to MCF envelopes are that these envelopes does not mediate infection of human cells. By displaying such an ecotropic trait, animal experiments involving viruses expressing a phenotype like the wild type SL3-2 envelope, can be performed under the lowest classification for genetically engineered cells or animals facility e.g. bio-safety level 1 according to Danish safety regulations.

One embodiment of the present invention relates to the use of a virus or vector according to present invention for gene discovery by infection of a new-born rodent.

In a presently preferred embodiments of the present invention, said rodent constitutively express the gag/pol genes of simple retroviruses, or said rodent express the gag/pol genes of simple retroviruses in a tissue specific manner, or the expression of the gag/pol genes of simple retroviruses is developmentally regulated said rodent, or said rodent express the gag/pol genes of gamma retroviruses both tissue specifically and in a developmentally regulated manner.

A particular embodiment of the present invention relates to a method for gene discovery comprising
a) using a virus or vector according to the present invention
b) infecting a new-born rodent with said virus or vector
c) inducing a tumour by means of said virus or vector
d) isolating said tumour in said rodent e) identifying a gene involved in the oncogenesis by cloning the integration site of said virus or vector in said tumour.

In a presently most preferred embodiment, the present invention relates to a method for gene discovery of cancer related genes.

### Safety

The degree to which laboratory security is implemented should be commensurate with risk. All laboratories, including those handling only low-risk biological materials under bio-safety level 1 containment practices, must maintain a basic level of security.

There are several blo-safety concerns that arise with the use of viral vectors including:

Tropism (host range) - viral vectors that can enter (infect) human cells are often used. Replication-deficient viral vectors can gain back the deleted genes required for replication (become replication-competent) through recombination - referred to as replication-competent virus (RCV) breakouts.

Genes may be expressed in tissues and/or organisms where they are normally not expressed. In the case of some genes such as oncogenes this could have far-reaching negative consequences.
When evaluating safety for use of viral vectors, there are a number of factors that need to be considered including risk group of the organism; tropism (organism and tissue); route of transmission; whether the virus integrates into the host genome; and the specific gene(s) being introduced.

Viral vectors frequently used are retrovirus/lentivirus, adenovirus, adeno-associated virus, poxvirus, herpes virus, and baculovirus. Amphotropic Moloney murine leukaemia virus (MMLV) and adenovirus are common viral vectors used to introduce genes into human cells.

NIH classifies amphotropic MMLV as RG2 pathogens, and thus MMLV viral vectors infect human cells and have potential for RCV breakthroughs. Amphotropic MMLV integrates into the host's genome and translates into stable expression of introduced genes and the potential for insertional mutagenesis of host genes.

There are a number of ways to improve safety when working with viral vectors e.g. to consider the alternative of nonviral vectors, limit tropism or use ecotropic MMLV with methods that allow the virus to enter human cells in a limited manner. This strategy is especially relevant for introduction of genes such as oncogenes, mutant tumor suppressor genes, mutant repair genes, and some signal transduction pathway genes.

Thus, a particular embodiment of the present invention relates to the use of any of the envelope polypeptides or vectors thereof having the properties of wild type SL3-2 in a bio-safety level 1/PS I/SI laboratory animal facilities or equivalents thereof.

By co-cultivation of two packaging cell lines each using a different receptor, it is possible to ping-pong the transducing vector back and forth between the two populations of packaging cells. As there will be no restriction to infection from a vector packaged in an envelope of the other packaging cell line this ping-pong effect of the vector will increase the number of vectors in the cells, and thereby increase the expression level of said vector.

By using a packaging cell line having the properties of wild type SL3-2 in conjunction with an ecotropic receptor, no virus particle will be made capable of infecting human cells and the experiment can thus be performed under the lowest level of classification regarding genetic modified cells or animals (e.g. class 1 according to Danish safety regulations).

As the skilled addressee will be aware, the polytropic/xenotropic receptors described in the present application have been described by various names within the literature such as, but not limited to Xpr1, Sxv, Rmc1, Syg1 and Rmc-1 for receptors of murine origin, whereas the human receptors are currently known as XPR1, X3, SYG1 and RMC1. Updated views over these locus sites are available via the Locus-Link web-tool on the NIH web-site at which the official gene symbol and name can be obtained.

### The mouse URL: <http://www.ncbi.nim.nih.gov/LocusLink/LocRpt.cgi?l=19775>

The human URL: <http://www.ncbi.nlm.nih.gov/LocusLink/LocRpt.cgi?l=9213>

### Legends to figures

### Figure 1

Alignment of amino acid sequences of selected MLVs in the VR3 region. Residues corresponding to arginine 212 are shown in the rectangular box.

### Figure 2

Amino acid aligning of SL3-2 and MCF 247 envelopes: The non-homologous regions of SU, including VR3 region, are marked by brackets.

### Figure 3

Depicted in panel A is a wild type replication competent virus.

In panel B said replication competent virus has an ScFv insert in the envelope for redirection of tropism.

Panel C and E are replication competent vectors where an heterologous translational cassette has been inserted into either the U3 region panel C or in the 3' un-translated region downstream of the envelope gene Panel E.

Panel D and F are the same as panel C and E except a ScFv has been inserted in the envelope for redirection of tropism.

Panel G is a replication competent retroviral vector expressing an envelope gene from a mono-cistronic mRNA by directing translation of the envelope gene by an internal ribosome entry site (IRES).

Panel H same as Panel G except that a ScFv has been inserted into the envelope gene for redirection of tropism. In the present context ScFv means a Single chain antibody or any other heterologous peptide sequence that mediates redirection of envelope tropism

### Figure 4

Library analysis of SU. An overlap extension reaction was used to create the DNA library, which is subsequently transfected into Plat E packaging cells. The packaging cells contain wild type envelope protein and therefore produce infectious particles independent of the mini-virus envelope genes. A stable population of infected semi-packaging cells (the cellular library) is established using these virions. The cells in the library produce virions that bear envelope mutants on the surface and contain the corresponding gene. Target cells are transduced and selected. The surviving colonies contained mini-viruses that encode functional envelopes.

### Figure 5

Semi-packaging cells containing a mini-virus vector produce virions. The mini-virus bearing virions can infect both semi-packaging cells and other permissive cells, but only semi-packaging cells are able to produce new virions. Mini-virus is thus conditional replication competent.

### Figure 6

The genomic structure of the mini-virus vector. All of the elements except *neo*, IRES and *env* are derived from Akv MLV.

### Examples

### Materials and methods

### Cells

Murine fibroblast cell line NIH 3T3 and NIH 3T3 derived packaging cell line Psi-2 (Mann et *al*., 1983) and semi-packaging cell line CeB (Cosset et *al.,* 1995) were grown in Dulbecco's modified Eagle's medium with glutamax-1 (Gibco BRL, Life Technologies) containing 10% (vol/vol) newborn calf serum, 100 U/mL penicillin and 100µg/mL streptomycin. The CeB semi-packaging cell line was selected with the same growth medium containing 40µg/mL blasticidin.

Human HeLa and TE 671 cells were grown under the same conditions as murine NIH 3T3 cells. Human HeLa mCAT (Aagaard *et al.,* 2002) cells were selected with the above described medium containing 75µg/mL Hygromycin B, and no blasticidin.

BOSC 23 packaging cells that are derived from the Ad5 transformed human embryonic kidney 293T cell line (Pear *et al.,* 1993) were selected with 250µg/mL xantine (Sigma), 25µg/mL mycophenolic acid (Gibco BRL, Life Technologies), 20µg/mL aminopterin (Sigma) and 60pg/mL thymidine (Sigma) in Dulbecco's modified Eagle's medium containing 10% (vol/vol) dialyzed fetal calf serum, 100 U/mL penicillin and 100µg/mL streptomycin.

The Plat E packaging cell line is also derived from human embryonic kidney 293T cell line (Morita *et al.,* 2000) and was selected with 10µg/mL blasticidin and 1µg/mL puromycin in Dulbecco's modified Eagle's medium with glutamax-1 (Gibco BRL, Life Technologies) containing 10% (vol/vol) fetal calf serum, 100 U/mL penicillin and 100µg/mL streptomycin. While not under selection, both Plat E and BOSC 23 cells were grown in Dulbecco's modified Eagle's medium with glutamax-1 (Gibco BRL, Life technologies) containing 10% (vol/vol) fetal calf serum, 100 U/mL penicillin and 100µg/mL streptomycin.

All cells were incubated at 37°C in 90% relative humidity and 5.7% CO₂.

### Transfections and transduction/titer measurements

Transfections were done by the calcium phosphate precipitation method without a glycerol shock (Graham *et al.,* 1973) and leaving the calcium precipitate on the cells for 24 hours.

Transfections of plasmids were done using 10µg of the plasmid in question and 1µg of an EGFP expressing plasmid (Clonetech). EGFP was included as a visual aid to determine the success of transfections.

Transfection of PCR products was achieved by adding to the approximately 300ng of PCR product 10µg of plasmid pUC19 as carrier DNA and 1µg of EGFP expressing plasmid.

Medium for the transfected cells was renewed every day until it was used for transduction, usually between 48 and 72 hours after transfection, depending on the number of green fluorescing cells. The day before transduction, medium was changed from bovine fetal serum to newborn calf serum containing medium.

Target cells were seeded at concentration of 5*10³ cells/cm² 24 hours before transduction. Supernatant from transfected cells was filtered (0.22µm). The supernatant with 6µg/mL polybrene was added either by 10-fold end-point dilution (to NUNC six-well-dishes for measuring titers) or crude (for creating pool of infected semi-packaging cells) to target cells, depending on the experiment.

24 hours later, the infected cells were selected using medium as described (DMEM with 10% NCS and 1% penicillin/streptavidin supplemented with 600 microgram G418 per ml) (Gibco BRL, Life Technologies, ) before containing 600mg/mL G418 for 10-12 days.

### Overlap extension reactions

### Downstream fragments

The Polymerase Chain Reactions (PCR) for synthesizing the overlap extension fragments were done using Expand High Fidelity PCR System (Roche) and 5ng template pr. 100µL reaction and 25 amplification rounds:

100µl reaction contained: 10µL 10 x buffer, 0.8mM dNTP (0.2mM of each nucleotide), 0.25pM of each primer and 2.625 units of enzyme, as above.

94°C: 2 min., 25 x (94°C: 1 min, 50°C: 1 min:, 72°C: 2 min. 30 sec.), 72°C: 7 min.

The primer (111019: AACAATTTCACACAGGAAACAGC) was used as the end primer in all reactions. Other primers used are shown in table 1.

**Table 1: Downstream primers**

| Library | Primer name | Primer Sequence |
|---|---|---|
| SL3-2/MCF VRA | B8142D01 | |
| SL3-2/MCF VRB | A0341A02 | |
| SL3-2/MCF VR3 | A0341A01 | |
| SL3-2/MCF Leader | B8142D02 | |
| SL3-2/MCF alternating VR3 library | A0571A05 | |
| SL3-2/MCF VR3 RT | T3130F07 | |
| SL3-2/MCF VR3 RI | T3130F08 | |
| SL3-2/MCF VR3 GT | T3130F09 | |
| SL3-2/MCF VR3 GI | T3130F10 | |

### Upstream fragments

The upstream fragments were made using the primer (111020:GATTAAGTTGGGT AAGCCAGGG) as the end primer. Other primers used are shown in table 2. PCR conditions were otherwise the same as those for downstream fragments.

**Table 2: Upstream primers**

| Construct | Primer name | |
|---|---|---|
| SL3-2/MCF VRA | B8087E02 | CACAGTGTGACCGGGGCAAAC |
| Sl3-2/MCF VRB | B8087E04 | AGGGAGTGTTTCCTCGCTTAAG |
| SL3-2/MCF VR3 | B8087E03 | GTCCCAGCTGGCCCTTTTACC |
| SL3-2/MCF Leader | B8087E05 | TAAGATTCCCAGGACTATTAG |

The fragments were purified from 1% agarose gels (by GFX PCR DNA and Gel Band Purification Kit, Amersham Pharmacia Biotech).

The overlap extension reaction was done using rTth DNA Polymerase XL (PE Biosystems) in ten rounds using end primers (111019 and 111020) and equimolar amounts of the two fragments:

100µL reaction contained: 30µL 3.3 XL buffer II, 1.375 mM Mg(OAc)₂, 0.8mM dNTP (0.2mM of each nucleotide), 0.25pM of each primer and 4 units of enzyme, as above.

95°C: 3 min., 10 x (95°C: 1 min., 60°C: 30 sec., 73°C: 6 min.), 73°C: 8 min.

The resulting overlap extension product was purified by Wizard DNA Clean-Up System (Promega) and used for transfection.

### Example 1 Generating the envelope protein of SL3-2

The envelope protein of SL3-2 was taken from genomic DNA of NIH 3T3 cells infected with SL3-2 virus. PCR was used to amplify the envelope. The upstream primer was chosen to match a conserved sequence upstream of the splice acceptor site among different MLV strains. The downstream primer was designed according to the known sequence of SL3-2 LTR (Dal *et al.,* 1990). The amplified PCR fragment was subsequently cloned into the mini-virus to replace the original Akv envelope. The new construct was designated NeoSL3-2mo. Three clones were chosen for sequencing.

One of the clones contained a frameshift mutation and was not infectious. This SL3-2 envelope shows a 92% homology on nucleotide level with the envelope protein of MCF-247 polytropic MLV. The latter has a wide host range and is able to infect cells from many species (Rein 1982) , (Hartley *et al.,* 1977), (Chattopadhyay et al.,1982), whereas the original reports claimed that SL3-2 has the same host range as the ecotropic viruses (Pedersen *et al.,* 1981), (Rein *et al.,* 1984).

### Cloning of SL3-2 envelope

The envelope of SL3-2 was amplified by the following PCR from the genomic DNA of infected NIH 3T3 cells.

### PCR conditions:

10µL 10 x buffer, 0.8mM dNTP (0.2mM of each nucleotide), 0.25pM of each primer and 2.625 units of enzyme (Expand High Fidelity PCR System (Roche)).

Using primers:
204820: CTCTCCAAGCTCACTTACAGGCCCTC
205585: TGCGGCCGCGTCGACTGGCTAAGCCTTATGAA

95°C: 2 min., 45 × (95°C: 1 min, 60°C: 30 sec., 73°C: 4 min.), 73°C: 5 min.

We have two different mini-virus vectors with Akv envelope that differ in the length of the spacer between the IRES element and *env* gene.
The Neo-env-mo vector contains a linker similar in size to the one found in wild type EMCV (Morgan et al. 1992, Bachrach et al.2002), while Bi-neo-env contains a longer linker with multiple cloning sites similar to the linker found in pJD214HyBi+ (Koo et al. 1992).

Spacer sequences in Bi-neo-env and Neo-env-mo are shown below. The ATG start codon of Env is shown in italics. The bold T (T) in the sequence corresponds to T827 in EMCV nucleotide coordinates (Jang and Wimmer 1990):
Bi-neo-env: AAACACGATTGCCGCGTGCGGCCGCTAACACTCCGGAGCTCGAGCCAAT*ATG*
Neo-env-mo: AAACACGAT-----AATACC*ATG*

The PCR fragment was purified from agarose gel (GFX PCR DNA and gelband gel purification kit from Amersham Pharmacia Biotech) and used to replace the fragment removed from pBiNeoEnv plasmid by NotI (Gibco BRL, Life Technologies) and CelII restriction enzymes (Amersham Lifescience). The new plasmid was named pBiNeoSL3-2. Subsequently, both pBiNeoSL3-2 and pNeoEnvmo were cut by NcoI (Gibco BRL, Life Technologies). The cut vector (pBiNeoSL3-2) was after dephosphorylation by shrimp alkaline phosphatase from Roche according to manufacture and ligated with the NcoI fragment of pNeoEnvmo resulting in the plasmid pNeoSL3-2mo.

Example 2 Determining host range and receptor usage of the new mini-virus To determine the host range of the cloned mini-virus, BOSC 23 packaging cells were transfected by two clones of NeoSL3-2mo. 24 hours later supernatant form these were used to transduce semi-packaging cells (CeB cells). CeB cells were selected for G418 resistance until they were confluent. The resistant semi-packaging cells contain the vector. Supernatant from these were used to measure the titer of vectors bearing SL3-2 envelope on a number of different cells. Murine NIH 3T3 and human HeLa cells were used. Psi-2 cells are packaging cells that express ecotropic envelope constitutively and thus block infection by ecotropic viruses. NIH 3T3 cells infected by MCF 247 block infection through polytropic receptor. HeLa cells that express ecotropic receptor mCAT-1 were also included to ensure that mini-virus is capable of infecting human cells

**Table 3:The tropism of SL3-2 mini-virus. Titers measured in cfu/mL.**

| Mini-virus bearing Env from | NIH3T3 | PSI-2 | NIH 3T3/ MCF 247 | HeLa mCAT | HeLa |
|---|---|---|---|---|---|
| Akv clone #1 | 1,6*10⁶ | 1,7*10² | 4*10⁵ | 5*10⁴ | 0 |
| Akv clone #2 | 8*10⁵ | 1,2*10² | 2,6*10⁴ | 3*10⁴ | 0 |
| SL3-2 clone #1 | 1,2*10⁵ | 1,7*10⁴ | <1 | 0 | 0 |
| SL3-2 clone #2 | 8*10⁵ | 6*10⁵ | 2 | 0 | <1 |

As can be seen in table 3, the tropism of SL3-2 mini-virus vectors bearing the Akv or SL3-2 envelopes has similar titers on NIH 3T3 cells. Expression of ecotropic envelope in PSI-2 cells lowers the titer of ecotropic mini-virus by a factor of around 10.000, but has no effect on the titer of SL3-2 bearing mini-virus. NIH 3T3/MCF 247 shows interference with SL3-2 envelope but not with that of Akv. The reason for lower interference levels seen in Psi-2 cells compared with MCF 247 infected NIH 3T3 cells is most likely that Psi-2 cells express ecotropic envelope less efficiently. This is not surprising since Psi-2 cells do not contain a replication competent virus. These data prove that SL3-2 uses polytropic and not the ecotropic receptor (mCAT-1). None of the envelopes in this study were able to mediate infection of human HeLa cells and the fact that HeLa cells that express ecotropic receptor are infected with mini-virus bearing the ecotropic envelope indicates that in the case of SL3-2 mini virus, infection is prohibited because of lack of envelope/receptor interactions. These results were confirmed in repeated experiments.

### Example 3 Determinants of the limited host range of SL3-2

Three regions showed amino acid differences between SL3-2 and MCF 247. Two of these regions correspond to parts of the variable VRA and VRB regions, whereas the third was a fifteen amino acids long stretch upstream of the proline rich region. To further investigate the determinants of the differences in tropism of these two viruses, chimeras were created in which these segments in SL3-2 were replaced by those of MCF 247, using the described overlap extension method (Jespersen et al., 1997).

Clone 1 of pNeoSL3-2mo was used in this study. As control, another chimera was created, in which the signal peptide of MCF 247 replaced that of SL3-2. There are two amino acid differences in the signal peptide, but since the signal peptide is not found in the mature envelope, no change of tropism was expected. The first three chimeras were named S/M Leader, S/M VRA and S/M VRB according to the segment that was changed. The construct in which the proline rich proximate region was changed was named S/M VR3.

These constructs were transfected into Plat E packaging (Morita et al., 2000). The produced virions were used to infect CeB semi-packaging cells. After selection of these for G418 resistance, titer of the mini-virus constructs were measured on NIH 3T3 murine cells, human TE 671 and HeLa cells, NIH 3T3 cells infected with MCF 247 and Psi-2 cells.

**Table 4: Receptor usage of SL3-2/MCF 247 chimeras. Titers measured in cfu/mL. ND: not determined.**

| | NIH 3T3 | NIH 3T3/ MCF 247 | Psi-2 | HeLa | TE 671 |
|---|---|---|---|---|---|
| S/M Leader | 2*10⁵ | 12,5 | 1*10⁵ | 0 | 5 |
| S/M VRA | 2,25*10⁵ | 2,5 | 4*10⁵ | 0 | 2,5 |
| S/M VRB | 3*10⁶ | 2,5 | 5*10⁵ | 0 | 5 |
| S/M VR3 | 5*10⁵ | 2,5 | 3*10⁵ | 2,5*10² | 2,25*10⁴ |
| NeoSL3-2mo | 3*10⁵ | 0 | 1*10⁵ | 0 | ND |

As can be seen in table 4, all of the constructs have comparable titers on NIH 3T3, MCF 247 infected NIH 3T3 and Psi-2 cells, indicating that all of the four chimeras utilize the same receptor with same efficiency in murine cells. Interestingly, the S/M VR3 construct had a 5,000 to 10,000 fold higher titer on human TE 671 cells than other constructs and was the only mini-virus capable of infecting human HeLa cells, indicating that a major determinant for utilizing the human receptor is to be found in this region.

Among the amino acids in the VR3 region, there are five that are different between SL3-2 and MCF 247. Any combination of these can be responsible for the different tropisms of these viruses.

To clarify this issue, a randomised library was created in which the amino acids found in the five positions alternated between those found in SL3-2 or MCF 247 by the described overlap extension method.

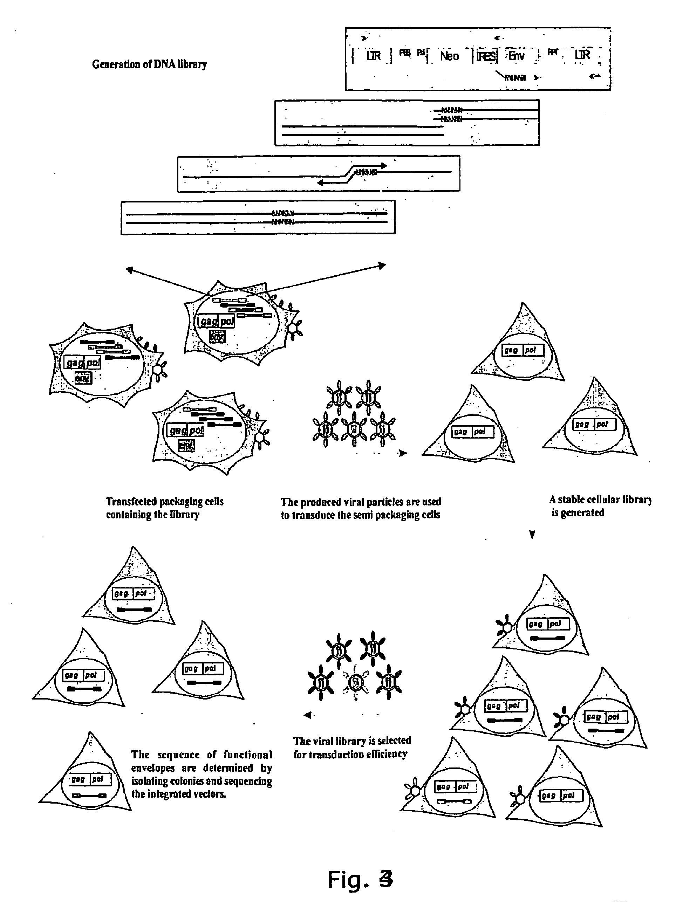

The product of the overlap extension reaction was transfected into Plat E packaging cells. These cells express Gag, Pol and Env proteins of Moloney MLV. Supernatant containing virions was harvested after 48 hours and used to transduce CeB semi-packaging cells. Infected cells were selected on the basis of their resistance to G418. This ensures that the populations of semi-packaging cells contain stably integrated vectors. CeB cells express moloney MLV Gag and Pol but not Env. Therefore any virions produced will carry the envelope protein encoded by the integrated vector. Since the same vector is packaged into the virion, the gene encoding the envelope protein will be integrated in the target cell, if the virion is infectious. Infected CeB cells were grown under G418 selection until they were confluent. Supernatant was harvested from these and used to transduce TE671 human cells seeded in petri-dishes (NUNC P10). Petri-dishes were chosen to facilitate isolation of colonies that would form. Colonies were isolated and the sequences of integrated vectors were determined. See figure 4. The library was designed so that each alternating amino acid could be translated from at least two different codons. This was done to distinguish any functional requirements of nucleotide sequence from those on the amino acid level. 34 colonies were isolated and sequenced.

The results are presented in table 5.

**Table 5: Colonies isolated from alternating SL3-2/MCF 247 VR3 library**

| Randomised positions | | | | | | | | | |
|---|---|---|---|---|---|---|---|---|---|
| SL3-2 | MCF 247 | SL3-2 | MCF 247 | SL3-2 | MCF 247 | SL3-2 | MCF 247 | SL3-2 | MCF 247 |
| Ala | Gly | Ser | Pro | Ala | Val | Arg | Gly | Thr | Ile |
| GCT Ala | | TCG Ser | | GTG val | | AGT Ser | | ATT Ile | |
| GCT Ala | | CCG Pro | | GTG Val | | GGG Gly | | ATC Ile | |
| GCT Ala | | TCC Ser | | GTA Val | | GGA Gly | | ATT Ile | |
| GGA Gly | | TCC Ser | | GTG Val | | GGA Gly | | ATC Ile | |
| GCT Ala | | TCA Ser | | GCA Ala | | GGA Gly | | ACC Thr | |
| GCT Ala | | CCA Pro | | GTG Val | | GGG Gly | | ACC Thr | |
| GCA Ala | | CCT Pro | | GCG Ala | | GGG Gly | | ATT Ile | |
| GCT Ala | | CCT Pro | | GCT Ala | | GGG Gly | | ATA Ile | |
| ACC Thr | | TCT Ser | | GCC Ala | | AGG Arg | | ACA Thr | |
| GGG Gly | | CCA Pro | | GCT Ala | | GGG Gly | | ATC Ile | |
| GCG Ala | | CCT Pro | | GTC Val | | GGA Gly | | ATT Ile | |
| GGA Gly | | TCC Ser | | GTG Val | | GGA Gly | | ATC Ile | |
| GGT Gly | | TCT Ser | | GTA Val | | GGG Gly | | ATC Ile | |
| ACG Thr | | TCC Ser | | GCG Ala | | GGA Gly | | ATT Ile | |
| GCT Ala | | TCG Ser | | GTT Val | | GGG Gly | | ATC Ile | |
| GCG Ala | | CCA Pro | | GCT Ala | | GGA Gly | | ACT Thr | |
| GCT Ala | | CCT Pro | | GCA Ala | | GGG Gly | | ATC Ile | |
| GGG Gly | | TCA Ser | | GTG Val | | GGG Gly | | ATC Ile | |
| GGT Gly | | CCG Pro | | GCT Ala | | GGA Gly | | ATT Ile | |
| AGT Ser | | TCC Ser | | GTC Val | | GGG Gly | | ATA Ile | |
| GCT Ala | | TCC Ser | | GCT Ala | | GGG Gly | | ATA Ile | |
| GGA Gly | | CCT Pro | | GCA Ala | | GGG Gly | | ATC Ile | |
| GGT Gly | | TCC Ser | | GTA Val | | GGA Gly | | ATC Ile | |
| GCC Ala | | TCG Ser | | GCT Ala | | GGA Gly | | ATT Ile | |
| GCA Ala | | CCT Pro | | GCG Ala | | GGG Gly | | ATT Ile | |
| GCG Ala | | TCG Ser | | GCT Ala | | GGA Gly | | ATT Ile | |
| GCT Ala | | TCT Ser | | GTG Val | | GGG Gly | | ATC Ile | |
| GGT Gly | | TCG Ser | | GTG Val | | GGA Gly | | ATA Ile | |
| GGT Gly | | CCT Pro | | GCC Ala | | GGG Gly | | ATT Ile | |
| GGTGly | | TCG Ser | | GTG Val | | GGG Gly | | ATA Ile | |
| AGC Ser | | TCG Ser | | GTA Val | | GGA Gly | | ATA Ile | |
| GCC Ala | | TCC Ser | | GCA Ala | | AGG Arg | | ACC Thr | |

| GCT Ala | | CCG Pro | | GCC Ala | | GGA Gly | | ATA Ile | |
|---|---|---|---|---|---|---|---|---|---|
| Ala | Gly | Ser | Pro | Ala | Val | Arg | Gly | Thr | Ile |
| 55% | 33% | 60% | 40% | 51% | 49% | 6% | 94% | 15% | 85% |

While the first three positions showed a more or less random choice of amino acids, there is a strong bias towards MCF's glycine and isoleucine in the fourth and fifth positions, suggesting that these two amino acids are the determinants for the different tropisms of SL3-2 and MCF 247 viruses. Random occurrence of codons confirmed that it is the protein sequence that is important for infection of human cells. A few isolated colonies contained amino acids other than those found in either SL3-2 or MCF 247. These probably result from inaccurate primer synthesis in primers used to create the library (the oligonucleotides do not have an equal distribution of the four or two nucleotides during incorporation of the NNK sequence of the primer).

### Example 4 Mutation studies

To confirm the results of the library study, four mutants were constructed, each containing a different combination of amino acids in the VR3 fourth and fifth non-homologous positions. The titers of these constructs were measured on murine NIH 3T3 and human TE 671 cells. The results are presented in table 6:

**Table 6 Titer of SL3-2 VR3 4th and 5th non-homologous position mutants**

| Mutated amino acids in 1^{st} and 2^{nd} positions | Titer (cfu/mL) on TE 671 cells | Titer (cfu/mL) on NIH 3T3 cells |
|---|---|---|
| RT (wild type) | 4.75 | 5 × 10⁶ |
| RI | 2.25 × 10¹ | 3.25 × 10⁶ |
| GT | 1.5 × 10³ | 2 × 10⁶ |
| GI | 4.5 × 10⁵ | 2 × 10⁶ |

All of the constructs have similar titers on murine cells suggesting correct expression and function of the envelopes. In agreement with the results of the library study, substituting both arginine and threonine of the SL3-2 VR3 with glycine and isoleucine of MCF 247 elevates the titer on human cells by a factor of around 100.000. Single substitutions yield envelopes with intermediate titers.

Substitution of either arginine or threonine yields envelopes with intermediate titers as shown in table 6, although substitutions of arginine had a more profound effect on tropism. We conclude that a glycine to arginine substitution in SL3-2 is the major determinant for limited tropism of this virus and that isoleucine to threonine has only a secondary effect.

### Example 4 Alignment of MLV envelopes

Alignment of MLV envelopes shows almost perfect homology in this region between polytropic, xenotropic and amphotropic sequences. It is possible that this segment plays an important role in mediating fusion by Env. Since the receptor for polytropic/xenotropic viruses differ from those for amphotropic viruses, the function of the VR3 segment might be independent of the receptor binding. This idea is confirmed by the fact that this region is outside the receptor binding domain of amphotropic and ecotropic envelopes (Battini *et al*., 1992), (Heard *et al.,* 1991).

In apparent disagreement, the entire N-terminal segment of polytropic and xenotropic envelopes including the (polytropic or xenotropic but not amphotropic) proline rich region is necessary for envelope function (Battini *et al.,* 1992), (Battini *et al.,* 1995). Polytropic and xenotropic N-terminal fragments of SU cannot confer superinfection resistance unless they also include the proline rich region, but the proline rich region neither binds the receptor on its own nor changes the tropism of the amphotropic envelope if it replaces amphotropic PRR.

These data suggest that the binding domain of polytropic and xenotropic envelopes are longer than that of ecotropic or amphotropic envelopes and also include the proline rich region. It is also possible that VR3 and proline rich region of polytropic and xenotropic envelopes are important in correct folding of the receptor binding domain and are not directly involved in binding the receptor.

The mutations in SL3-2 only affect the infection of human cells, while the mouse cells are infected effectively.

### Example 5 The mini-virus system

Mini-virus system consists of a vector, expressing a marker gene and envelope, and of a complementing cell line, designated a semi-packaging cell line, expressing Gag and Pol proteins. Expression of envelope is achieved by inserting an IRES element from EMCV between the marker and *env* genes. The major difference between the mini-virus system and conventional vector systems is that in mini-virus envelope is expressed from the transfer vector whereas in other systems, it is expressed from the packaging cells.

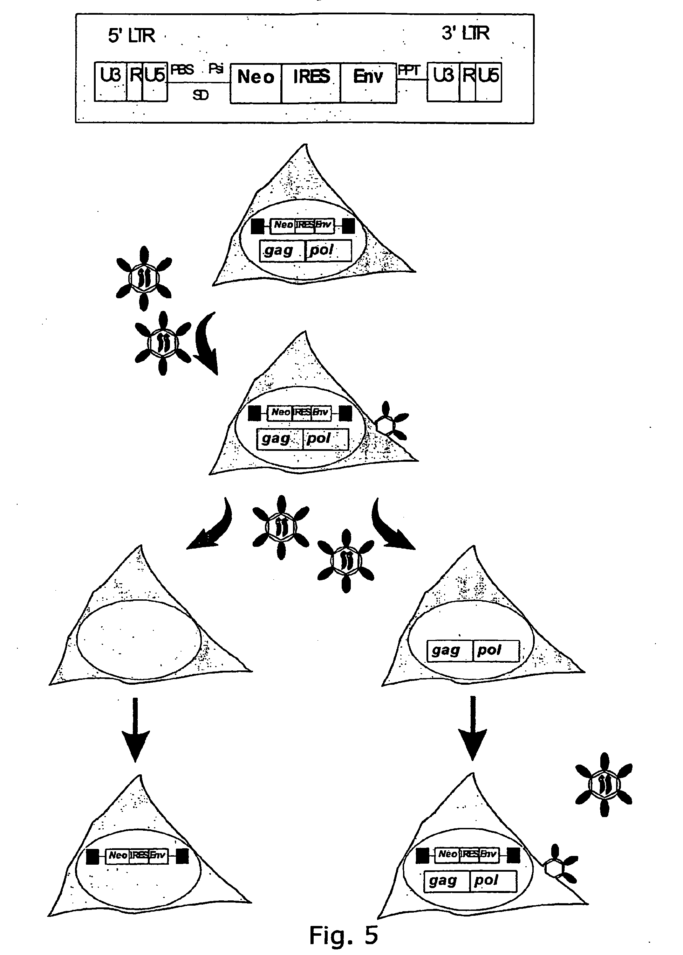

Expressing envelope from the transfer vector instead of the packaging cells has two major advantages. First superinfection resistance in packaging cell line is avoided. In traditional packaging cell lines, envelope is expressed constitutively, resulting in superinfection resistance. Hence a virus using the same receptor cannot infect these cells. This makes it impossible for a vector to spread in a culture of packaging cells. In the mini-virus system this is avoided by expressing envelope from the vector. The mini-virus is thus replication competent in the semi-packaging cell line (that expresses Gag and Pol), but not in other cells. Secondly, since the virions carry the *env* gene, the phenotype of the envelope protein can be directly linked to the genotype of a particular *env* gene, just as studies with wild type viruses. See figure 5.

These properties, and the fact that infected cells can be easily selected using the marker gene, makes this system very suitable for studying envelope proteins. Random mutational analysis can be done, in which envelopes with particular characteristics can be selected from a large pool and their primary structure deduced from the sequence of integrated vector in the cellular genome. The relatively small size of mini-virus makes creation of random mutations in specific parts of *env* possible by PCR based methods (Jespersen *et al*., 1997). Alternatively, evolutionary studies of envelope can be done, in which selection of the marker gene can drive *in vitro* evolution of the envelope protein.

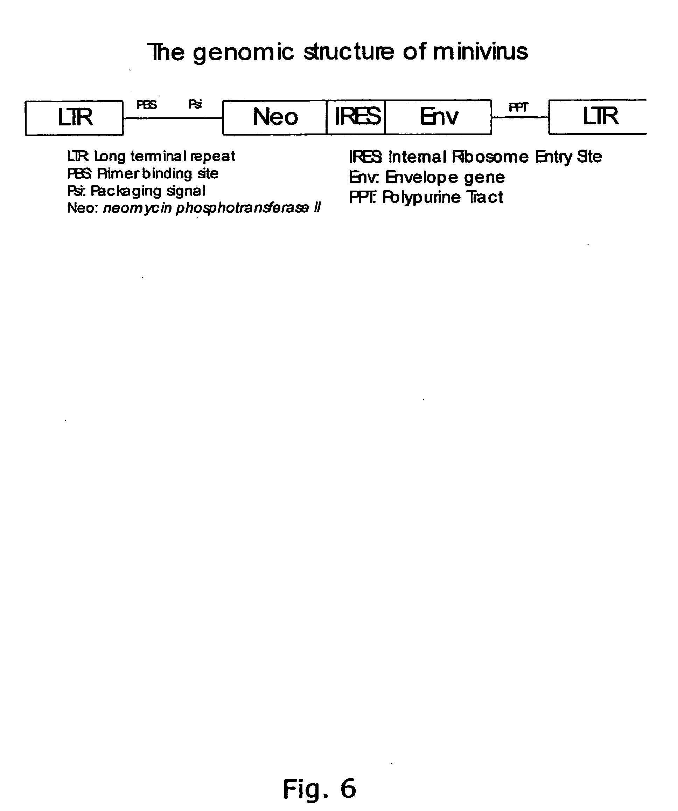

The vector employed in this study Neo-env-mo (see figure 6) is based on Akv MLV (Van Beveren *et al.,* 1985) and contains IRES element derived from ECMV (Morgan *et al.,* 1992) and the *neomycin phosphotransrease II* (Beck *et al*., 1982) (*neo*) as the marker gene.

The *neo* gene confers upon mammalian cells resistance to the aminoglycoside antibiotic G418, which otherwise blocks protein synthesis.

The overlap extension method (Jespersen *et al.,* 1997) was used to create the mutants and the randomised libraries. This method benefits from the fact that introduction of alternative or random nucleotides into ends of a PCR-amplified DNA fragment pool is possible if the primers used are designed accordingly. To introduce such randomisation inside a vector, two fragments, one of which contains the randomised sequence, are synthesised using PCR. The fragments are designed so that they span the whole vector when combined and at the same time share an overlapping sequence at their ends. The two fragments can act as primers for each other in a subsequent PCR by virtue of the overlapping sequence. The second PCR thus reproduces the vector that contains the randomised sequence. To increase the yield, end primers were added to amplify the overlap extension product as it is formed. See figure 6.

### Example 6 Mutation studies

To investigate the role of residues 212 and 213 in determining the tropism of SL3-2 envelope, a library in which these two positions were randomised was created. Unlike the library explained in example 3, in which only two amino acids were possible at each randomised position, this library (in future referred to as the NNK library) allowed all 20 different amino acids at these two positions. The randomised codons only contained G or T at the third position to minimize the occurrence of stop codons from 3 to 1, while allowing at least one codon for each amino acid. The library was generated, transfected and selected in the same way as explained in example 3. The results are presented in table 7.

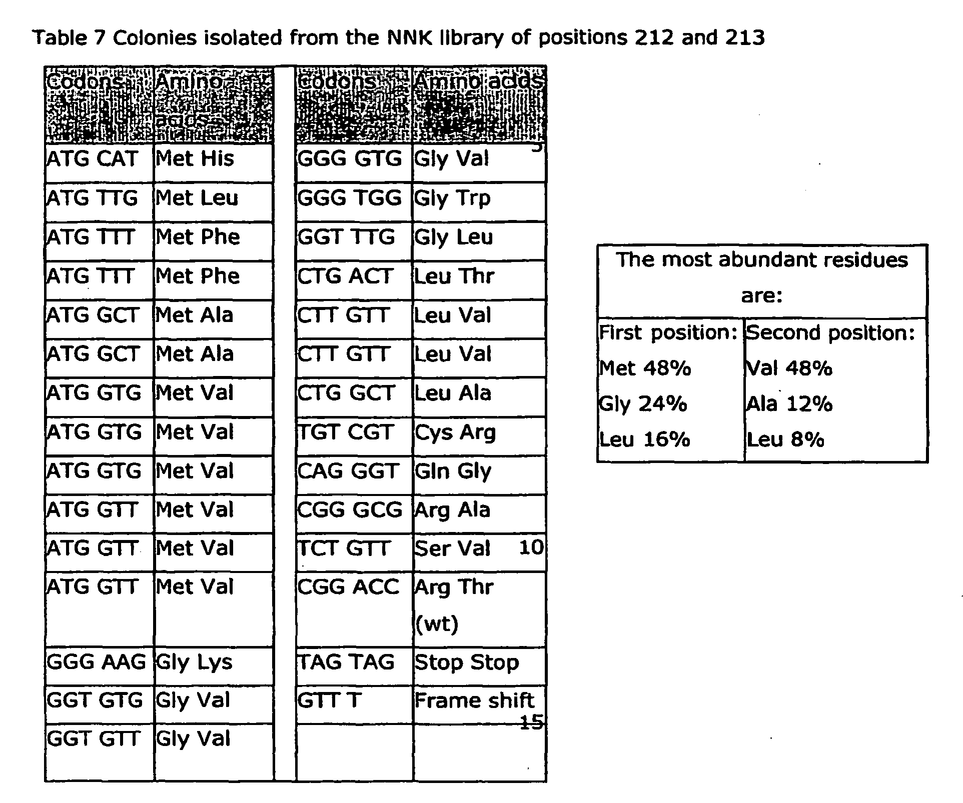

Few amino acids were represented in the selected colonies. Most noteworthy was the fact that around half of the colonies contained Methionine at position 212, likewise, half of the colonies contained valine at position 213. If the effects of methionine and valine on tropism is independent of each other, it is expected that around a fourth (½ x ½) of the isolated colonies should contain both methionine and valine. This is indeed the case as seen in Table 7.

Roughly equal occurrence of different available codons for valine (GTG or GTT) confirms that the overrepresentation of valine at position 213 in isolated colonies is a result of functional selection and not a bias in the library.

Two of the isolated colonies contained sequences that result in production of truncated envelope proteins. One contained two consecutive stop codons and the other a deletion of two nucleotides resulting in frameshift.

Such incidents probably occur when a semi-packaging cell in the cellular library, which is initially infected by a vector containing the defective envelope gene, is reinfected by a vector encoding a functional envelope. When this happens, the semi-packaging cell would be able to produce infectious viral particles, using the functional envelope gene, which package the vector with defective envelope gene. Thus a limited number of non-functional envelope mutants might be isolated from a library.

Re-infection of cells usually does not happen since the viral receptors on the cellular surface interact with the surface-bound envelope protein and are not available to external viruses, a phenomenon known as superinfection resistance. In the case of truncated envelope proteins, no such interaction is obviously possible, hence reinfection can be expected.

15 mutants, in which amino acids at positions 212 and 213 were chosen according to the results of the NNK library study or occurrence in wt polytropic/xenotropic viruses, were made. Titer of these constructs were measured on murine NIH 3T3, Human TE 671 and mink CCL-64 cells. As control, a mini-virus expressing the envelope protein of MCF 247 was used. Results are shown in Table 8.

**Table 8: Titers (c.f.u./ml of selected mutants from 2aa NNK library**

| Envelope backbone | Aa nr 212 | Aa nr 213 | NIH 3T3 Murine | TE 671 Human | CCL 64 Mink |
|---|---|---|---|---|---|
| SL3-2 | R | T (wt) | 7.2 × 10⁶ | < 4 | 1.6 × 10¹ |
| SL3-2 | R | I | 6.4 × 10⁶ | 2.4 × 10¹ | 2.4 × 10² |
| SL3-2 | R | A | 1 × 10⁷ | 4 | 2.4 × 10¹ |
| SL3-2 | G | T | 8.4 × 10⁶ | 5.2 × 10² | 5.2 × 10⁴ |
| SL3-2 | **G** | **I** | **7.6 × 10⁶** | **4,4 × 10⁵** | **2.4 × 10⁶** |
| SL3-2 | G | V | 3.6 × 10⁶ | 3.2 × 10⁵ | 2.4 × 10⁵ |
| SL3-2 | G | A | 5.6 × 10⁶ | 2.8 × 10³ | 2 × 10⁴ |
| SL3-2 | G | K | 5.2 × 10⁶ | 5.6 × 10³ | 2 × 10⁵ |
| SL3-2 | L | I | 2.4 × 10⁶ | 1.2 × 10³ | 2 × 10⁴ |
| SL3-2 | L | A | 1.1 × 10⁷ | 5.2 × 10³ | 1.6 × 10³ |
| SL3-2 | L | V | 3.6 × 10⁶ | 1.6 × 10⁴ | 2.4 × 10⁴ |
| SL3-2 | **M** | **V** | **9.6 × 10⁶** | **7,2 × 10⁶** | **2.8 × 10⁶** |
| SL3-2 | M | I | 2.4 × 10⁵ | 2.8 × 10⁴ | 3.2 × 10⁴ |
| SL3-2 | M | A | 9.6 × 10⁶ | 2.5 × 10⁵ | 2.5 × 10⁵ |
| SL3-2 | C | R | 2.4 × 10⁶ | 1.2 × 10¹ | 1.2 × 10² |
| **MCF 247 (wt)** | **G** | **I** | **4.8 × 10⁶** | **1.5 × 10²** | **1.2 × 10⁵** |

The mutant with methionine at position 212 and valine at position 213 has the highest titer on human cells in accordance with the findings in the NNK library study. Methionine and valine are not found at these positions in any wild type viruses. The MV mutant shows over 10 fold increase in titer when compared to the GI mutant.

Glycine and Isoleucine are found in a number of naturally occurring polytropic viuses including MCF 247. Interestingly, the SL3-2 mutants show a remarkably higher titer on human cells compared to MCF 247 mini-virus, suggesting other parts of the envelope are involved in tropism determination.

In SL3-2 mutants, the titer on mink cells correspond roughly to the titer on the human cells, although in some cases the titer on mink cells is 10-100 fold higher. Interestingly, MCF 247 is a 1000 times more efficient in infecting mink cells than human cells.

### Example 7 SL3-2 mutants and not SL3-2 wild type can infect the different human cell types described here

The tropism of selected SL3-2 mutants were tested on two other human cell lines to ensure that the function of positions 212 and 213 in the VR3 region are not cell specific.

**Table 9 Titers on different human cell lines**

| Mutant | Titer on NIH 3T3 cells | Titer on TE 671 cells | Titer on CCL-64 Mink cells | Titer on Human Hela cells | Titer on 293 cells |
|---|---|---|---|---|---|
| SL3-2 wt (RT) | 7.2 × 10⁶ | < 2 | 1.6 × 10¹ | <2 | <2 |
| SL3-2(GI) | 7.6 × 10⁶ | 4.4 × 10⁵ | 2.4 × 10⁶ | 1.3 × 10² | 2 × 10⁵ |
| SL3-2(LI) | 2.4 × 10⁶ | 1.2 × 10³ | 2 × 10⁴ | ND | 1.2 × 10⁴ |
| SL3-2(MV) | 9.6 × 10⁶ | 7.2 × 10⁶ | 2.8 × 10⁶ | 1.0 × 10² | 2.4 × 10⁵ |
| MCF 247 | 4.8 × 10⁶ | 1.5 × 10² | 1.2 × 10⁵ | 2 | 2.4 × 10³ |

As can be seen in the table 9, SL3-2 wt has undetectable or negligible titer on all the three human cell lines tested. Mutation of residue 212 to methionine or glycine and residue 213 to valine or isoleucine has a profound but variable effect on the ability of SL3-2 envelope to infect human cells. The SL3-2 mutants much less efficient in infecting the human HeLa cells, still there is a significant increase in the titer when compared to the wild type SL3-2. Titers on 293 cells are comparable although somewhat less than those on TE 671 cells.

Example 8 SL3-2 mutants utilise the polytropic receptor on human cells.

Tropism change of the SL3-2 mutants could be accompanied by a shift in receptor usage. To investigate if the SL3-2 mutants utilise the polytropic receptor on human cells, TE 671 cells were transfected by wt MCF 247 pro-viral DNA and linearized pPUR plasmid, which expresses the puromycin resistance gene in mammalian cells, using the calcium phosphate precipitation method (Graham, F. L., and van der Eb, A. J., 1973). The cells were subsequently selected with 2.5 µg/mL of puromycin until single colonies arose.

The colonies were isolated and sorted according to surface envelope expression by FACS using the anti-envelope antibody (83A25) with 89-99% efficiency.

The titer of the mutants were subsequently measured on envelope expressing as well as wt TE 671 cells.

**Table 9 Superinfection resistance assay**

| Mutant | Titer on NIH 3T3 cells | Titer on TE 671 cells | Titer on TE 671 cells transfected with wt MCF 247 |
|---|---|---|---|
| SL3-2 wt (RT) | 7.2 × 10⁶ | <2 | ND |
| SL3-2(GI) | 7.6 × 10⁶ | 4.4 × 10⁵ | 6 × 10³ |
| SL3-2(LI) | 2.4 × 10⁶ | 1.2 × 10³ | 6.8 × 10¹ |
| SL3-2(MV) | 9.6 × 10⁶ | 7.2 × 10⁶ | 1.6 × 10⁴ |
| MCF 247 | 4.8 × 10⁶ | 1.5 × 10² | 1.6 × 10¹ |

As can be seen in table 9, the titer of all three tested SL3-2 mutants is decreased by almost 100 fold as a result of superinfection resistance by the endogenous MCF envelope expression suggesting that the SL3-2 mutants utilise the same receptor (Rmc1) as the MCF virus.

Superinfection resistance is not absolute since only 98-99% of the target cells express the MCF envelope, as determined by flow cytometry analysis. In accordance a 100 fold reduction in titers is observed (only one in 100 cells can be infected).

### Example 9 Position 199

Among the amino acids in the VR3 region, there are five that are different between SL3-2 and MCF 247.

As described previously (example 3), a randomised library has been created in which the amino acids found in these five positions of the VR3 region of SL3-2 envelope alternated between those found in SL3-2 or MCF 247 using the described overlap extension method.

The product of the overlap extension reaction was transfected into Plat E packaging cells. These cells express Gag, Pol and Env proteins of Moloney MLV. Supernatant containing virions was harvested after 48 hours and used to transduce CeB semi-packaging cells.

Infected cells were selected on the basis of their resistance to G418. This ensures that the populations of semi-packaging cells contain stably integrated vectors. CeB cells express Moloney MLV Gag and Pol but not Env. Therefore, any virions produced will carry the envelope protein encoded by the integrated vector. Since the same vector is packaged into the virion, the gene encoding the envelope protein will be integrated in the target cell, if the virion is infectious. Infected CeB cells were grown under G418 selection until they were confluent. Supernatant was harvested from these and used to transduce TE671 human cells seeded in petri-dishes (NUNC P10). Petri-dishes were chosen to facilitate isolation of colonies that would form. Colonies were isolated and the sequences of integrated vectors were determined. See figure 4. The library was designed so that each alternating amino acid could be translated from at least two different codons. This was done to distinguish any functional requirements of nucleotide sequence from those on the amino acid level. 34 colonies were isolated and sequenced.

The results are presented in table 5.

Several of the mutants isolated from this library contained a threonine or the closely related serine in the first randomised position, position 199. The design of the library only allowed codons in the randomised positions that encode amino acids found in either SL3-2 (alanine) or MCF 247 (glycine) envelopes.

The randomised codons originate from the randomised primers used in the overlap extension method to create the library. Hence, occurrence of the unexpected threonine and serine at this position probably originates from an error in the primer synthesis and/or the PCR procedure.

In either case, the subset of mutants that contained the unintended Thr and Ser has probably been much smaller than those that contained valine or glycine.

Thus, appearance of serine and threonine at the first randomised position could represent a positive selection of these two amino acids from a small pool of mutants. Two different codons have been found for both serine and threonine, implicating a genuine functional selection at the protein level instead of a bias towards a unique sequence in the DNA library.

Presumably, threonine or serine at position 199 has a positive effect on the ability of the polytropic envelope family to infect non-murine and specifically human cells.

The present inventors are testing this hypothesis by creating mutants containing threonine at position 199 and either SL3-2 or MCF 247 sequences in the rest of the VR3 region by overlap extension method explained previously. Titer of these mutants on none murine cells will be compared to the titer of either SL3-2 wt envelope or the SL3-2 VR3 mutant (as described in table 4). Any effect of threonine at position 199 on tropism of SL3-2 virus would be reflected in an increase and/or decrease of the titer of the mutant compared to the titer of wt SL3-2. Ukewise, any effect of threonine at position 199 on tropism of the VR3 SL3-2 mutant (table 4) can be detected in titer differences between the VR3 SL3-2 mutant and VR3 SL3-2 mutant containing threonine at position 199.

### References

Aagaard L, Mikkelsen JG, Warming S, Duch M, Pedersen FS., 2002, J. Gen. Virol., 83:439-42
Battini J.L., Heard J.M., and Danos O., 1992, J. Virol. 66, 1468-1475.
Battini J-L., Danos O., and Heard J.M., 1995, J. Virol. 69, 713-719.Bachrach, E., M. Pelegrin, M. Piechaczyk, F. S. Pedersen, and M. Duch. 2002. Virology 293, 328-334.
Beck E., Ludwig G., Auerswald E.A., Reiss B., and Schaller H., 1982, Gene 19, 327-336.
Chattopadhyay S.K., Cloyd M.W., Linemeyer D.L., Lander M.R., Rands E., and Lowy D.R., 1982, Nature 295, 25-31
Cosset F-L., Takeuchi Y., Battini J.L., Weiss R.A., and Collins M.K., 1995, J. Virol. 69, 7430-7436.
Dai H.Y., Etzerodt M., Baekgaard A.J., Lovmand S., Jorgensen P., Kjeldgaard N.O., and Pedersen F.S.,1990, Virology 175, 581-585.
Golan TJ and Green-MR 2002. J. Virol 76(7): 3558-63.
Graham F.L., van der Eb A.J., 1973, Virology 52, 456-467.
Hartley, J.W., Wolford N.K., Lloyd J.O., and Rowe W.P., 1977, Proc.Natl. Acad. Sci. USA. 74, 789-792.
Heard J.-M., and Danos O., 1991, J. Virol. 65, 4026-4032.
Jang, S. K., and E. Wimmer. 1990. Genes Dev. 4,1560-1572.
Jespersen T., Duch M., and Pedersen F.S.,1997, Biotechniques 23,48-52.
Koo, H. M., A. M. C. Brown, R. J. Kaufman, C. M. Prorock, Y. Ron, and J. P. Dougherty. 1992. Virology 186, 669-675.
Mann R., Mulligan R.C., Baltimore D., 1983, Cell 33, 183-189.
Morgan R.A., Couture L., Elroy-Stein O., Ragheb J., Moss B., and Anderson W.F., 1992, Nucleic Acid Rec. 20, 1293-1299.
Morita S., Kojima T., and Kitamura T., 2000, Gene Ther. 7, 1063-1066.
Pear W.S., Nolan G.P., Scott M.L., and Blatimore D., 1993, PNAS 90, 8392-8396.
Pedersen, F.S., Crowther R.L., Tenney D.Y., Reimold A.M., and Hasletine W.A., 1981, Nature 292, 167-170.
Rein A., 1982, Virology 120, 251-257
Rein A., and Schultz A., 1984, Virology 136, 144-152.Towers et al 1999. PNAS vol. 97, no. 22 pp12295-12299
Van Beveren C., Coffin J., and Hughes S., 1985, RNA tumour viruses.CSHL Press, New York, 790-805.

## Claims

1. A purified retroviral envelope polypeptide comprising an amino acid sequence which is at least 94% identical to the amino acid sequence shown in SEQ ID NO: 2, or a fragment of said amino acid sequence that is at least 94% identical to the sequence shown in SEQ ID NO: 2, wherein said fragment comprises the VR3 region, which comprises all of the amino acids found between residue found at two positions after the conserved tryptophan 197 of SEQ ID NO:2 and the residue before the conserved aspartic acid 214 SEQ ID NO:2, including said two positions, wherein said polypeptide is capable of mediating infection of a cell by use of the polytropic/xenotropic receptor encoded by the Rmc1 locus of the NIH Swiss inbred NFS/N mouse for entry and unable of mediating infection of a cell by use of a human polytropic/xenotropic receptor encoded by the human RMC1 locus or
a purified retroviral envelope polypeptide comprising an amino acid sequence which is at least 94% identical to the amino acid sequence shown in SEQ ID NO: 2, or a fragment of said amino acid sequence that is at least 94% identical to the sequence shown in SEQ ID NO: 2, wherein said polypeptide is capable of mediating infection of a human cell and wherein said polypeptide includes at least one substitution in the VR3 region, which comprises all of the amino acids found between residue found at two positions after the conserved tryptophan 197 of SEQ ID NO:2 and the residue before the conserved aspartic acid 214 SEQ ID NO:2, including said two positions.

2. The purified retroviral envelope polypeptide according to claim 1, wherein said mutation is at position 212 in SEQ ID NO: 2.

3. The purified retroviral envelope polypeptide according to claim 1 or 2, wherein said at least one substitution alters the host tropism of a virus or an infectious particle comprising said polypeptide.

4. A purified retroviral envelope polypeptide according to any of claims 1-3, wherein said purified polypeptide is a murine retroviral envelope polypeptide capable of mediating infection of a human cell.

5. A purified retroviral envelope polypeptide according to any of claims 1 to 4, wherein said mutation at position 212 in SEQ ID: 2 results in a methionine.

6. A purified retroviral envelope polypeptide according to any of claims1-5 capable of mediating a higher infectivity in human cells than MCF-247, MCF-13 and X-MLV (NZB) viruses.

7. A purified retroviral envelope polypeptide according to any of claims 1-6, further comprising an inserted non-viral sequence capable of redirecting the target cell specificity, by the resultant chimeric envelope.

8. A purified retroviral envelope polypeptide according to any of claim 7, wherein said inserted sequence is a single chain antibody.

9. A purified retroviral envelope polypeptide according to any of claims 1-8, further comprising a chemical modification of said envelope.

10. A purified retroviral envelope polypeptide according to claim 9, wherein said chemical modification enhances and/or alters the host tropism.

11. A recombinant mammalian cell displaying an envelope polypeptide according to any of claims 1-10.

12. An isolated nucleic acid sequence encoding any of the envelope polypeptides according to any of claims 1-10.

13. The isolated nucleic acid sequence according to claim 12 as shown in SEQ ID NO: 1

14. A recombinant mammalian expression vector comprising a nucleic acid sequence coding for envelope polypeptide according to claims 1-6 and/or 7-10.

15. A replication competent retrovirus, comprising a purified envelope polypeptide according to any of claims 1-6 and/or 7-10.

16. The replication competent retrovirus according to claim 15 further comprising a heterologous translation cassette.

17. A retrovirus according to claim 16, wherein said heterologous translation cassette consists of an IRES-gene element.

18. A retroviral expression vector comprising a nucleic acid sequence coding for envelope polypeptide according to claims 1 and/or 7-10

19. A retroviral expression vector comprising a nucleic acid sequence coding for envelope polypeptide according to claims 2-6 and/or 7-10.

20. A vector according to claims 14 or 18-19, further comprising at least one heterologous gene to be expressed.

21. A vector according to claim 20, wherein expression of the envelope is directed by a IRES-element.

22. A packaging cell construct comprising a recombinant mammalian expression vector comprising a nucleic acid coding for an envelope polypeptide according to any of claims 1-10, and a non-viral or viral promoter and poly-adenylation signals.

23. Use of a vector according to any of claims 14-22, for the generation of a packaging cell.

24. The use according to claim 23 for expression in a cell constitutively expressing the gag/pol genes of simple retroviruses.

25. Use of a packaging cell according to any of claims 22-24 for the preparation of a composition for the modification of a cell.

26. The use according to claims 23 and/or 24, for the preparation of a composition for the modification of a cell.

27. The use according to claim 23, or of a replication competent retrovirus comprising a purified envelope polypeptide according to any of claims 1 and/or 7 to 10 for gene discovery by infection of a new-born rodent.

28. The use of a virus or vector according to claim 27, wherein said rodent constitutively express the gag/pol genes of simple retroviruses.

29. Use of a virus or vector according to claim 27, wherein said rodent express the gag/pol genes of simple retroviruses in a tissue specific manner.

30. Use of a virus or vector according to claim 27, wherein said rodent expression of the gag/pol genes of simple retroviruses is developmentally regulated.

31. Use of a virus or vector according to claim 27, wherein said rodent expresses the gag/pol genes of gamma retroviruses tissue specifically and in a developmentally regulated manner.

32. A method for gene discovery comprising
a) using a virus or vector according to any of claims 14-21 or a replication competent retrovirus comprising an envelope polypeptide according to any of claims 1 and/or 7-10;
b) infecting a new-born rodent with said virus or vector
c) inducing a tumour by means of said virus or vector
d) isolating said tumour in said rodent
e) identifying a gene involved in the oncogenesis by cloning the integration site of said virus or vector in said tumour.

33. A method according to claim 38 for gene discovery of a cancer related gene.

34. Use of any of the envelope polypeptides according to claims 1 and/or 7-10 or vectors comprising said polypeptides.

## Patentansprüche

1. Gereinigtes retrovirales Hüllpolypeptid, umfassend eine Aminosäuresequenz, die zu der in SEQ ID NO: 2 gezeigten Aminosäuresequenz mindestens 94 % identisch ist, oder ein Fragment der Aminosäuresequenz, die zu der in SEQ ID NO: 2 gezeigten Sequenz mindestens 94 % identisch ist, worin das Fragment die VR3 Region umfasst, die alle die Aminosäuren umfasst, die zwischen einem Rest gefunden werden, der nach dem konservierten Tryptophan 197 von SEQ ID NO: 2 und dem Rest vor der konservierten Asparaginsäure 214 von SEQ ID NO: 2, an zwei Positionen gefunden werden, einschließlich der zwei Positionen, worin das Polypeptid eine Infektion einer Zelle vermitteln kann, in dem der polytrophe/xenotrophe Rezeptor, der durch den Rmc1 Locus der NIH Swiss Inzucht NFS/N Maus kodiert wird, zum Eintritt verwendet wird und eine Infektion einer Zelle nicht vermitteln kann, in dem ein polytrophe/xenotrophe Rezeptor eines Menschen, der durch dem RMC 1 Locus des Menschen kodiert wird, verwendet wird, oder
ein gereinigtes retrovirales Hüllpolypeptid, das eine Aminosäuresequenz umfasst, die zu der in SEQ ID NO: 2 gezeigten Aminosäuresequenz mindestens 94 % identisch ist, oder ein Fragment der Aminosäuresequenz, die zu der in SEQ ID NO: 2 gezeigten Sequenz mindestens 94 % identisch ist, worin das Polypeptid eine Infektion einer menschlichen Zelle vermitteln kann und worin das Polypeptid mindestens eine Substitution in der VR3 Region einschließt, die alle die Aminosäuren umfasst, die zwischen einem Rest gefunden werden, der nach dem konservierten Tryptophan 197 von SEQ ID NO:2 und dem Rest vor der konservierten Asparaginsäure 214 von SEQ ID NO: 2, an zwei Positionen gefunden werden, einschließlich der zwei Positionen.

2. Gereinigtes retrovirales Hüllpolypeptid nach Anspruch 1, worin die Mutation an Position 212 in SEQ ID NO: 2 vorliegt.

3. Gereinigtes retrovirales Hüllpolypeptid nach Anspruch 1 oder 2, worin die mindestens eine Substitution den Wirtstropismus eines Virus oder eines infektiösen Partikels, das das Polypeptid umfasst, ändert.

4. Gereinigtes retrovirales Hüllpolypeptid nach einem der Ansprüche 1-3, worin das gereinigte Polypeptid ein Hüllpolypeptid eines Retrovirus der Maus ist, das eine Infektion einer Zelle des Menschen vermitteln kann.

5. Gereinigtes retrovirales Hüllpolypeptid nach einem der Ansprüche 1-4, worin die Mutation an Position 212 in SEQ ID NO:2 zu einem Methionin führt.

6. Gereinigtes retrovirales Hüllpolypeptid nach einem der Ansprüche 1-5, das in menschlichen Zellen eine höhere Infektiosität als MCF-247-, MCF-13- und X-MLV (NZB) Viren vermittelt.

7. Gereinigtes retrovirales Hüllpolypeptid nach einen der Ansprüche 1-6, weiter umfassend eine eingefügte nicht virale Sequenz, die die Spezifität für die Zielzelle durch die resultierende chimäre Hülle umschalten kann.

8. Gereinigtes retrovirales Hüllpolypeptid nach Anspruch 7, worin die eingefügte Sequenz ein Einzelketten-Antikörper ist.

9. Gereinigtes retrovirales Hüllpolypeptid nach einem der Ansprüche 1-8, das weiterhin eine chemische Modifikation der Hülle umfassend.

10. Gereinigtes retrovirales Hüllpolypeptid nach Anspruch 9, worin die chemische Modifikation den Wirtstropismus verbessert und/oder ändert.

11. Rekombinante Säugetierzelle, die ein Hüllpolypeptid nach einem der Ansprüche 1-10 zeigt.

12. Isolierte Nukleinsäuresequenz, die ein Hüllpolypeptid nach einem der Ansprüche 1-10 kodiert.

13. Isolierte Nukleinsäuresequenz nach Anspruch 12, die in SEQ ID NO:2 gezeigt ist.

14. Rekombinanter Säugetier-Expressionsvektor, umfassend eine Nukleinsäuresequenz, die ein Hüllpolypeptid nach einem der Ansprüche 1-6 und/oder 7-10 kodiert.

15. Replikationskompetenter Retrovirus, umfassend ein gereinigtes Hüllpolypeptid nach einem der Ansprüche 1-6 und/oder 7-10.

16. Replikationskompetenter Retrovirus nach Anspruch 15, der weiterhin eine heterologe Translationskassette umfasst.

17. Retrovirus nach Anspruch 16, worin die heterologe Translationskassette aus einem IRES Genelement besteht.

18. Retroviraler Expressionsvektor, umfassend eine Nukleinsäuresequenz, die ein Hüllpolypeptid nach einem der Ansprüche 1 und/oder 7-10 kodiert.

19. Retroviraler Expressionsvektor, umfassend eine Nukleinsäuresequenz, die ein Hüllpolypeptid nach einem der Ansprüche 2-6 und/oder 7-10 kodiert.

20. Vektor nach einem der Ansprüche 14 oder 18-19, der weiterhin mindestens ein zu exprimierendes heterologes Gen umfasst.

21. Vektor nach Anspruch 20, worin die Expression der Hülle durch ein IRES Element geregelt wird.

22. Verpackungszellkonstrukt, umfassend einen rekombinanten Säugetier-Expressionsvektor, der eine Nukleinsäure eines Hüllpolypeptids nach einem der Ansprüche 1-10 und einen nicht viralen oder viralen Promotor und Polyadenylierungssignale kodiert.

23. Verwendung eines Vektors nach einem der Ansprüche 14-22 zur Herstellung einer Verpackungszelle.

24. Verwendung nach Anspruch 23 für eine Expression in einer Zelle, die die gag/pol Gene einfacher Retroviren konstitutiv exprimiert.

25. Verwendung einer Verpackungszelle nach einem der Ansprüche 22-24 für die Herstellung einer Zusammensetzung für die Modifizierung einer Zelle.

26. Verwendung nach Anspruch 23 und/oder 24 für die Herstellung einer Zusammensetzung für die Modifikation einer Zelle.

27. Verwendung nach Anspruch 23, oder eines replikationskompetenten Retrovirus, umfassend ein gereinigtes Hüllpolypeptid nach einem der Ansprüche 1 und/oder 7 bis 10 für die Gen-Auffindung durch Infektion eines neugeborenen Nagetiers.

28. Verwendung eines Virus oder Vektors nach Anspruch 27, worin das Nagetier die gag/pol Gene einfacher Retroviren konstitutiv exprimiert.

29. Verwendung eines Virus oder Vektors nach Anspruch 27, worin das Nagetier die gag/pol Gene einfacher Retroviren in einer gewebespezifischen Weise exprimiert.

30. Verwendung eines Virus oder Vektors nach Anspruch 27, worin die Nagetierexpression der gag/pol Gene einfacher Retroviren entwicklungsgemäß reguliert ist.

31. Verwendung eines Virus oder Vektors nach Anspruch 27, worin das Nagetier die gag/pol Gene von gamma Retroviren gewebespezifisch und in einer entwicklungsgemäßen regulierten Weise exprimiert.

32. Verfahren zur Gen-Auffindung, umfassend:
a) Verwenden eines Virus oder Vektors nach einem der Ansprüche 14-21 oder eines replikationskompetenten Retrovirus, der ein Hüllpolypeptid nach einem der Ansprüche 1 und/oder 7-10 umfasst,
b) Infizieren eines neugeborenen Nagetiers mit dem Virus oder Vektor,
c) Induzieren eines Tumors durch das Virus oder den Vektor,
d) Isolieren des Tumors in dem Nagetier,
e) Identifizieren eines Gens, das an der Onkogenese beteiligt ist, durch Klonieren der Integrationsstelle des Virus oder Vektors in dem Tumor.

33. Verfahren nach Anspruch 32 zum Gen-Auffinden eines mit Krebs in Verbindung stehenden Gens.

34. Verwendung eines Hüllpolypeptides nach Ansprüchen 1 und/oder 7-10 oder Vektoren, die diese Polypeptide umfassen.

## Revendications

1. Polypeptide purifié de l'enveloppe rétrovirale comprenant une séquence d'acides aminés qui est au moins identique à 94 % avec la séquence d'acides aminés représentée par SEQ ID NO : 2, ou un fragment de ladite séquence d'acides aminés qui est au moins identique à 94 % avec la séquence représentée par SEQ ID NO : 2, où ledit fragment comprend la région VR3, qui comprend la totalité des acides aminés trouvés entre le résidu trouvé au niveau de deux positions après le tryptophane 197 conservé de SEQ ID NO : 2 et le résidu avant l'acide aspartique 214 conservé de SEQ ID NO : 2, y compris lesdites deux positions, où ledit polypeptide est capable de médier l'infection d'une cellule par l'utilisation du récepteur polytrope/xénotrope codé par le locus Rmc1 de la souris NFS/N congénique NIH Swiss pour l'entrée et incapable de médier l'infection d'une cellule par l'utilisation d'un récepteur polytrope/xénotrope humain codé par le locus RMC1 humain ou
polypeptide purifié de l'enveloppe rétrovirale comprenant une séquence d'acides aminés qui est au moins identique à 94 % avec la séquence d'acides aminés représentée par SEQ ID NO : 2, ou un fragment de ladite séquence d'acides aminés qui est au moins identique à 94 % avec la séquence représentée par SEQ ID NO : 2, où ledit polypeptide est capable de médier l'infection d'une cellule humaine et où ledit polypeptide comprend au moins une substitution dans la région VR3, qui comprend la totalité des acides aminés trouvés entre le résidu trouvé au niveau de deux positions après le tryptophane 197 conservé de SEQ ID NO : 2 et le résidu avant l'acide aspartique 214 conservé de SEQ ID NO : 2, y compris lesdites deux positions.

2. Polypeptide purifié de l'enveloppe rétrovirale selon la revendication 1, où ladite mutation est au niveau de la position 212 dans SEQ ID NO : 2.

3. Polypeptide purifié de l'enveloppe rétrovirale selon la revendication 1 ou 2, où ladite au moins une substitution modifie le tropisme envers l'hôte d'un virus ou d'une particule virale comprenant ledit polypeptide.

4. Polypeptide purifié de l'enveloppe rétrovirale selon l'une quelconque des revendications 1 à 3, où ledit polypeptide purifié est un polypeptide de l'enveloppe de rétrovirus murin capable de médier l'infection d'une cellule humaine.

5. Polypeptide purifié de l'enveloppe rétrovirale selon l'une quelconque des revendications 1 à 4, où ladite mutation au niveau de la position 212 dans SEQ ID NO : 2 produit une méthionine.

6. Polypeptide purifié de l'enveloppe rétrovirale selon l'une quelconque des revendications 1 à 5, capable de médier une infectivité supérieure dans des cellules humaines par rapport aux virus MCF-247, MCF-13 et X-MLV (NZB).

7. Polypeptide purifié de l'enveloppe rétrovirale selon l'une quelconque des revendications 1 à 6, comprenant en outre une séquence non virale insérée capable de rediriger la spécificité des cellules cibles, par l'enveloppe chimérique résultante.

8. Polypeptide purifié de l'enveloppe rétrovirale selon la revendication 7, où ladite séquence insérée est un anticorps à chaîne unique.

9. Polypeptide purifié de l'enveloppe rétrovirale selon l'une quelconque des revendications 1 à 8, comprenant en outre une modification chimique de ladite enveloppe.

10. Polypeptide purifié de l'enveloppe rétrovirale selon la revendication 9, où ladite modification chimique amplifie et/ou modifie le tropisme envers l'hôte.

11. Cellule mammalienne recombinante présentant un polypeptide de l'enveloppe selon l'une quelconque des revendications 1 à 10.

12. Séquence d'acide nucléique isolé codant pour l'un quelconque des polypeptides de l'enveloppe selon l'une quelconque des revendications 1 à 10.

13. Séquence d'acide nucléique isolé selon la revendication 12, telle que représentée par SEQ ID NO : 1.

14. Vecteur d'expression mammalien recombinant comprenant une séquence d'acide nucléique codant pour un polypeptide de l'enveloppe selon les revendications 1 à 6 et/ou 7 à 10.

15. Rétrovirus compétent pour la réplication, comprenant un polypeptide purifié de l'enveloppe selon l'une quelconque des revendications 1 à 6 et/ou 7 à 10.

16. Rétrovirus compétent pour la réplication selon la revendication 15, comprenant en outre une cassette de traduction hétérologue.

17. Rétrovirus selon la revendication 16, où ladite cassette de traduction hétérologue est constituée d'un élément de gène IRES.

18. Vecteur d'expression rétroviral comprenant une séquence d'acide nucléique codant pour le polypeptide de l'enveloppe selon les revendications 1 et/ou 7 à 10.

19. Vecteur d'expression rétroviral comprenant une séquence d'acide nucléique codant pour le polypeptide de l'enveloppe selon les revendications 2 à 6 et/ou 7 à 10.

20. Vecteur selon les revendications 14 ou 18 à 19, comprenant en outre au moins un gène hétérologue à exprimer.

21. Vecteur selon la revendication 20, où l'expression de l'enveloppe est dirigée par un élément IRES.

22. Produit de construction de cellule d'encapsidation comprenant un vecteur d'expression mammalien recombinant comprenant un acide nucléique codant pour un polypeptide de l'enveloppe selon l'une quelconque des revendications 1 à 10 et un promoteur non viral ou viral et des signaux de polyadénylation.

23. Utilisation d'un vecteur selon l'une quelconque des revendications 14 à 22, pour la génération d'une cellule d'encapsidation.

24. Utilisation selon la revendication 23, pour une expression dans une cellule exprimant constitutivement les gènes gag/pol de rétrovirus simples.

25. Utilisation d'une cellule d'encapsidation selon l'une quelconque des revendications 22 à 24, pour la préparation d'une composition destinée à la modification d'une cellule.

26. Utilisation selon les revendications 23 et/ou 24, pour la préparation d'une composition destinée à la modification d'une cellule.

27. Utilisation selon la revendication 23, ou d'un rétrovirus compétent pour la réplication comprenant un polypeptide purifié de l'enveloppe selon l'une quelconque des revendications 1 et/ou 7 à 10 pour la découverte de gènes par infection d'un rongeur nouveau-né.

28. Utilisation d'un virus ou d'un vecteur selon la revendication 27, où ledit rongeur exprime constitutivement les gènes gag/pol de rétrovirus simples.

29. Utilisation d'un virus ou d'un vecteur selon la revendication 27, où ledit rongeur exprime les gènes gag/pol de rétrovirus simples d'une manière spécifique du tissu.

30. Utilisation d'un virus ou d'un vecteur selon la revendication 27, où ladite expression du rongeur des gènes gag/pol de rétrovirus simples est régulée de manière développementale.

31. Utilisation d'un virus ou d'un vecteur selon la revendication 27, où ledit rongeur exprime les gènes gag/pol de gamma-rétrovirus de manière spécifique du tissu et d'une manière régulée par le développement.

32. Procédé de découverte de gènes comprenant
a) l'utilisation d'un virus ou d'un vecteur selon l'une quelconque des revendications 14 à 21 ou d'un rétrovirus compétent pour la réplication comprenant un polypeptide de l'enveloppe selon l'une quelconque des revendications 1 et/ou 7 à 10 ;
b) l'infection d'un rongeur nouveau-né avec ledit virus ou vecteur ;
c) l'induction d'une tumeur au moyen dudit virus ou vecteur ;
d) l'isolement de ladite tumeur dans ledit rongeur ;
e) l'identification d'un gène impliqué dans l'oncogenèse en clonant le site d'intégration dudit virus ou vecteur dans ladite tumeur.

33. Procédé selon la revendication 32, pour la découverte d'un gène associé à un cancer.

34. Utilisation de l'un quelconque des polypeptides de l'enveloppe selon les revendications 1 et/ou 7 à 10 ou des vecteurs comprenant lesdits polypeptides.
